# EUROPEAN PATENT SPECIFICATION

(11) **EP 4 173 567 B1**
(45) Date of publication and mention of the grant of the patent: **10.12.2025**
(21) Application number: 22204227.7
(22) Date of filing: 27.10.2022
(51) Int. Cl.: A61B 5/11, A61B 3/113, A61B 5/398, A61B 5/00

(54) **METHOD FOR DETECTING AN ACTIVITY OF EYES OF A USER OF GLASSES**
VERFAHREN ZUR ERKENNUNG EINER AUGENAKTIVITÄT EINES BRILLENBENUTZERS
PROCÉDÉ DE DÉTECTION D'UNE ACTIVITÉ OCULAIRE D'UN UTILISATEUR DE LUNETTES

(30) Priority: 29.10.2021 IT 202100027866
(43) Date of publication of application: 03.05.2023
(73) Proprietor: STMicroelectronics S.r.l., 20864 Agrate Brianza (MB) (IT)
(72) Inventor: ALESSI, Enrico Rosario, 95127 CATANIA (IT); PASSANITI, Fabio, 96100 SIRACUSA (IT)
(74) Representative: Studio Torta S.p.A.

(56) References cited:
- EP-A1- 3 760 116
- US-A1- 2021 217 286
- KANOGA SUGURU ET AL: "Assessing the effects of voluntary and involuntary eyeblinks in independent components of electroencephalogram", NEUROCOMPUTING, ELSEVIER, AMSTERDAM, NL, vol. 193, 10 February 2016 (2016-02-10), pages 20 - 32, XP029509079, ISSN: 0925-2312, DOI: 10.1016/J.NEUCOM.2016.01.057

## Description

The present invention relates to a method for detecting an activity of eyes of a user of glasses. In particular, it relates to a detection method based on the use of electrodes configured to detect electrostatic charge variations generated by movements of the eyes, to a pair of glasses thereof comprising the electrodes and a control unit configured to implement the detection method, and to a computer program product thereof.

As known, electrooculography (EOG) is a technique to measure the corneo-retinal potential (CRP) that exists between the front portion (e.g., comprising the cornea) and the back portion (e.g., comprising the retina) of the human eye. In fact, the eye acts as a dipole where the front pole at the cornea is positive and the back pole at the retina is negative.

EOG is a commonly used measure as CRP is higher than the potentials involved in electroencelography (EEG signals), since the eye is placed outside the skull and therefore there is no bone structure that attenuates the electrical signal generated. To measure the eye movement, pairs of electrodes are placed around the eye and in contact with the skin of the face, and for example a pair of electrodes above and below the eye and a pair of electrodes to the left and right of the eye.

In greater detail, when the eye rotates around an own center of rotation (e.g., substantially central to the same eyeball), it generates a CRP variation which is detected through the electrodes. In particular, when the eyelid closes the cornea moves in one direction while when the eyelid opens the cornea moves in the opposite direction, and this generates a CRP variation that is clearly recognizable and associable with this blink. The signal resulting from a blink generally has a low frequency, for example comprised between about 1 Hz and about 13 Hz**.**

Involuntary blinks have been shown to be correlated to a person's mental state, and in particular they are indicative of cognitive states such as relaxation or attention. For example, the Inter-Eye blink interval (IEBI) is a known and reliable biomarker of the degree of concentration of a person intent on performing an activity that requires visual attention, such as watching a film or performing manual work.

The detection of movements of the eyes, such as blink, may be relevant for applications aimed at determining the attention level of the user, the risks resulting from sleep and fatigue, visual disturbances or neurodegenerative diseases, or more simply to automatically activate functions in mobile and portable devices. In fact, detecting the movements of the eyes is relevant in applications such as smart glasses where knowing the direction of the gaze of the person who is wearing them is important, as well as acquiring commands provided by the person (e.g., voluntary blink, etc.). For example, eye tracking may activate advanced functions for adjusting the focal point of a camera lens or quick reading functions such as zoom and panoramic operations.

However, this known measurement technique suffers from the following problems: measurement noise due to alternating electric current at 50 Hz or 60 Hz possibly present in the environment where the measurement is carried out; noise due to head and body movements during the measurement; artifacts in the measurement caused by the operation of electrical apparatuses present in proximity to the electrodes; measurement errors due to contraction of the facial or neck muscles, or to the slipping of the electrodes on the skin due to sweat and eyelid blink.

Document EP 3 760 116 A1 generally relates to an eye blink sensor, to a method of examining blinking of an eye of a user and to a computer program. In particular, the eye blink sensor comprises three electrodes (two for signal detection and one for establishing a common ground), thus is relatively costly. Moreover, these electrodes are placed in contact with the skin of the user, thus their measures are subject to factors such as the moisture of the user's skin and the electrical signals travelling through the user's facial muscles. Furthermore, this document relates to a measure performed only on one eye of the user and does not implement eyeball tracking features.

The aim of the present invention is to provide a method for detecting the activity of eyes of a user of glasses, said glasses and a computer program product that overcome the drawbacks of the prior art.

According to the present invention, a method for detecting the activity of eyes of a user of glasses, said glasses and a computer program product are provided, as defined in the annexed claims.

For a better understanding of the present invention, preferred embodiments are now described, purely by way of non-limiting example, with reference to the attached drawings, wherein:
- Figure 1 is a perspective view of a pair of glasses comprising first electrostatic charge variation sensors, according to an embodiment;
- Figure 2 is a block diagram schematically illustrating the glasses of Figure 1;
- Figure 3 schematically shows two electrodes of one of the first electrostatic charge variation sensors of Figure 1, relative to an eye of a user exemplarily shown in three different positions;
- Figure 4 is a perspective view of a different embodiment of the glasses comprising the first electrostatic charge variation sensors and second electrostatic charge variation sensors;
- Figure 5 is a block diagram of a method for detecting an activity of eyes of a user of the glasses of Figure 1, according to an embodiment;
- Figures 6-9 and 13 are graphs of electrical signals acquired through the first and second electrostatic charge variation sensors of Figures 1 and 4;
- Figures 10-12 are respective block diagrams of respective and further embodiments of the method for detecting the activity of eyes of the user of the glasses of Figure 1 or 4.

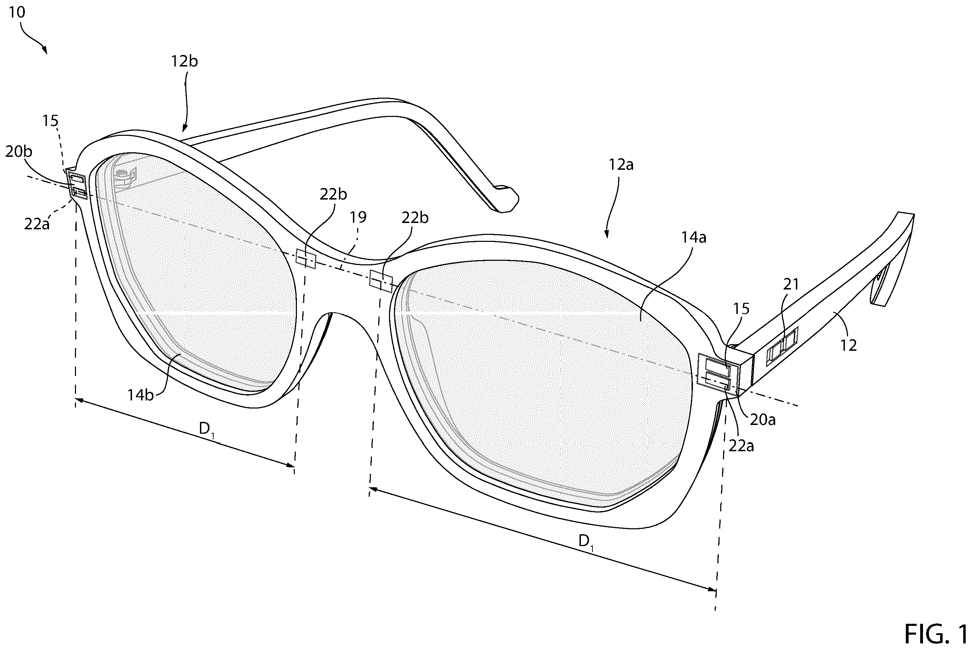

Figure 1 shows a pair of glasses 10 wearable on a user's face (not shown). In particular, the glasses 10 comprise a frame 12 (optional) having a first and a second support portion 12a and 12b (e.g., of annular shape) which support and/or accommodate a first and, respectively, a second lens 14a and 14b. For example, the glasses 10 may be prescription or sun glasses, or be smart glasses.

The glasses 10 further comprise a main control unit 21 and one or more first electrostatic charge variation sensors electrically coupled to the main control unit 21 and fixed to the frame 12, and therefore arranged in proximity to the eyes of the user when the latter wears glasses 10.

According to an exemplary embodiment, the main control unit 21 (such as a microprocessor, a microcontroller or a dedicated calculation unit) comprises, coupled to each other, a processing unit 21a and a data storage unit 21b (such as a memory, e.g. a non-volatile memory) for storing the acquired data. For example, the main control unit 21 is integrated into the frame 12.

In the embodiment exemplarily considered hereinafter and shown in Figure 1, a first left electrostatic charge variation sensor and a first right electrostatic charge variation sensor (shown in Figure 1 with the respective references 20a and 20b) carried by the first and, respectively, by the second support portions 12a and 12b are exemplarily considered; however, the number of first electrostatic charge variation sensors may be greater (e.g., two or more first electrostatic charge variation sensors for each of the support portions 12a and 12b).

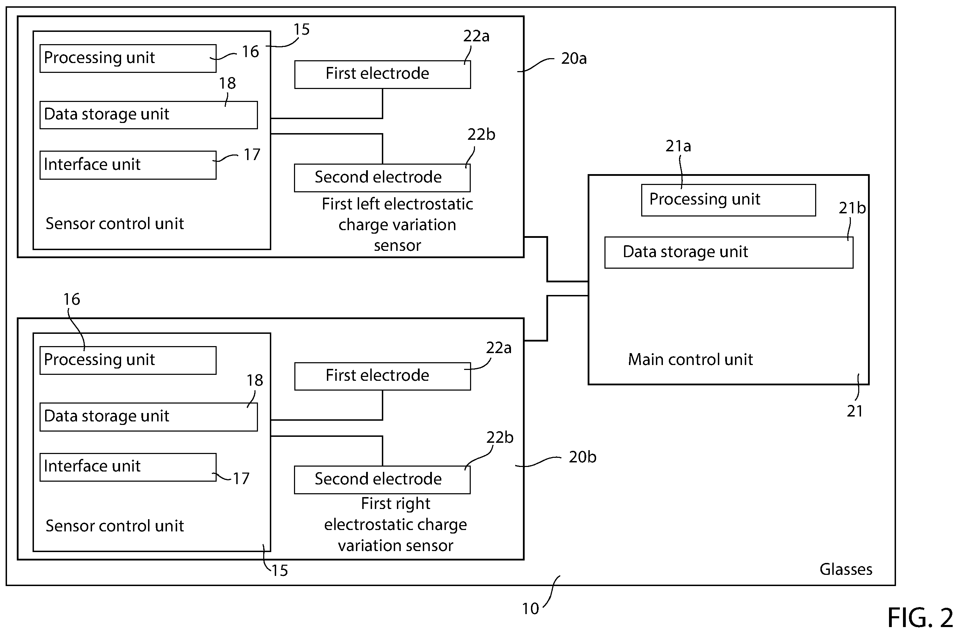

In particular and as better shown in Figure 2, each first electrostatic charge variation sensor 20a, 20b comprises a sensor control unit 15 and two or more electrodes which are spaced from each other, are fixed to the frame 12 and are electrically coupled to the sensor control unit 15. In the embodiment exemplarily considered hereinafter and shown in Figure 1 and 2, a first and a second electrode shown with the respective references 22a and 22b for each first electrostatic charge variation sensor are exemplarily considered; however, the number of electrodes for each first electrostatic charge variation sensor 20a, 20b may be greater (e.g., four electrodes).

In use, each electrode 22a, 22b detects a respective electrostatic charge variation caused by movements of the eyes of the user, as better discussed below, and generates a respective detection signal S_{R} indicative of said electrostatic charge variation.

In detail, each electrode 22a, 22b may have a metal surface or be totally metal coated by dielectric material, or even have a metal surface arranged under an external case of the glasses 10. In any case, during use, each electrode 22a, 22b is electrostatically coupled to the environment having the glasses 10 present therein, and in greater detail to the user's eye which is closest to said electrode 22a, 22b, in order to detect the induced electrostatic charge variation thereof.

According to an embodiment, each electrode 22a, 22b is integrated into the external case of the glasses 10, and for example comprises a conductive track formed on, or in, a semiconductor material wafer comprised in the glasses 10. According to a different embodiment, each electrode 22a, 22b is a metal element present in the glasses 10. Optionally, when a possible use of the glasses 10 in a humid environment (more specifically in water) is envisaged, each electrode 22a, 22b is inserted inside a waterproof case or in any case it is shielded by one or more protective layers, so as to prevent direct contact of the electrode 22a, 22b with water or humidity: in this case, the waterproof case or the one or more protective layers are of a material (e.g., dielectric or insulating material, such as plastics) such that it does not shield the electrostatic charge generated by the user's eye, which needs to be acquired by the electrode 22a, 22b. Other embodiments are possible, as apparent to the person skilled in the art, so that the electrodes 22a, 22b are electrostatically coupled to the user's eyes during use.

Furthermore, according to an exemplary embodiment, the sensor control unit 15 (such as a microprocessor, a microcontroller or a dedicated calculation unit) comprises, coupled to each other: an interface unit 17 (optional and of known type) electrically coupled to the electrodes 22a and 22b to interface the latter with the sensor control unit 15 (e.g., the interface unit 17 comprises an amplification circuit and/or an analog-to-digital converter, ADC, not shown); a respective processing unit 16 for processing detection signals S_{R} acquired through the electrodes 22a and 22b (and optionally processed through the interface unit 17); and a respective data storage unit 18 (such as a memory, e.g. a non-volatile memory) for storing the acquired data. For example, the sensor control unit 15 is integrated into the respective electrostatic charge variation sensor 20a, 20b.

In detail, each sensor control unit 15 is configured to process (in a per se known manner, for example by amplifying and converting into digital) the respective detection signals S_{R} acquired through the electrodes 22a and 22b and to generate a respective first electrostatic charge variation signal S_{Q,1} indicative of a difference between the detection signals S_{R} acquired through the first and second electrodes 22a and 22b. In particular, the first electrostatic charge variation signal S_{Q,1} is of digital type and is indicative of a difference between the electrostatic charge variations detected through the electrodes 22a and 22b.

According to an embodiment, the first and second electrodes 22a and 22b of each first electrostatic charge variation sensor 20a, 20b are spaced from each other. In particular, the electrodes 22a and 22b have a first mutual distance D₁ from each other and, for example, are arranged on respective opposite ends of the respective support portion 12a, 12b (e.g., they are diametrically opposite to each other with respect to the respective support portion 12a, 12b of annular shape). For example, the first and second electrodes 22a and 22b are aligned with each other along a first axis 19 which joins the first and second lenses 14a and 14b (e.g., which joins the centers, e.g. the barycenter, of the lenses 14a and 14b).

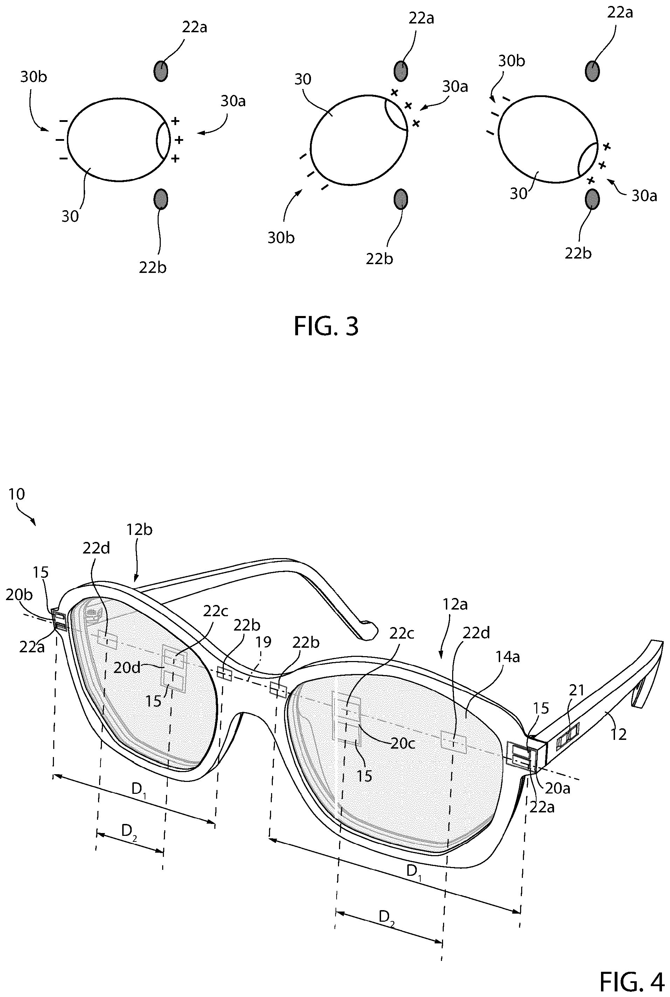

In other words, as schematically shown in Figure 3, the first and second electrodes 22a and 22b are arranged so that they detect the movements of the eye (indicated in Figure 3 with the reference 30, and comprising eyelids and an eyeball extending in an orbital cavity) of the user when the latter wears the glasses 10, and for example face opposite sides of the cornea (indicated in Figure 3 with the reference 30a) and, in greater detail, opposite sides of the pupil.

According to an embodiment shown in Figure 4, the glasses 10 further comprise one or more second electrostatic charge variation sensors similar to the first electrostatic charge variation sensors 20a and 20b and therefore not further described. In the embodiment exemplarily considered hereinafter and shown in Figure 4, a second left electrostatic charge variation sensor and a second right electrostatic charge variation sensor (shown in Figure 1 with the respective references 20c and 20d) carried by the first and, respectively, by the second lenses 14a, 14b are exemplarily considered; however, the number of second electrostatic charge variation sensors may be smaller or greater.

Each second electrostatic charge variation sensor 20c, 20d comprises respectively, instead of the first and the second electrodes 22a and 22b, a third and a fourth electrode 22c and 22d which are spaced both from each other and with respect to the first and second electrodes 22a and 22b and which are similar to the first and second electrodes 22a and 22b (and therefore are not described again in detail). In particular, the third and fourth electrodes 22c and 22d are radially internal with respect to the first and second electrodes 22a and 22b with respect to the center of the respective lens 14a, 14b. For example, the third and fourth electrodes 22c and 22d have a second mutual distance D₂ from each other that is less than the first mutual distance D₁ and, for example, are fixed to the respective lens 14a, 14b so as to face the eye 30 of the user when the latter wears the glasses 10. For example, the third and fourth electrodes 22c and 22d are aligned with each other and with the first and second electrodes 22a and 22b along the first axis 19, such that the third and fourth electrodes 22c and 22d are interposed between the first and second electrodes 22a and 22b. In other words, as schematically shown in Figure 4, when the user wears the glasses 10, the third and fourth electrodes 22c and 22d are arranged so as to detect the movements of the eye 30 and for example one faces the lacrimal caruncle and the other faces the opposite end of the eye 30 with respect to the lacrimal caruncle. In this embodiment, each sensor control unit 15 is further configured, similarly to what has been previously described for the first and second electrodes 22a and 22b, to process (in a per se known manner, for example by amplifying and converting into digital) the respective detection signals S_{R} acquired through the third and fourth electrodes 22c and 22d and to generate a respective second electrostatic charge variation signal S_{Q,2} indicative of a difference between the detection signals S_{R} acquired through the third and fourth electrodes 22c and 22d. In particular, the second electrostatic charge variation signal S_{Q,2} is of digital type and is indicative of a difference between the electrostatic charge variations detected through the third and fourth electrodes 22c and 22d.

In general, since the eye 30 operates as an electric dipole with a positive pole at the cornea 30a and a negative pole at the retina (indicated in Figure 3 with the reference 30b), the movements of the eye 30 generate electric field variations in the environment surrounding the eye 30, and these electric field variations induce variations in the induced electrostatic charge which are detectable through the electrodes 22a and 22b (and through the electrodes 22c and 22d, if any). Since the electrodes 22a and 22b (and the electrodes 22c and 22d, if any) are physically and electrically separated from each other, they are at different distances from each other with respect to the cornea 30a and the retina 30b and therefore detect electrostatic charge variations which differ from each other. This allows a differential detection of the electrostatic charge variations, as better discussed below. This allows both the movements of the eyes in the absence of blink and the eyelash blinks (or eyelid blinks) of the user to be detected, as it has been shown that each blink corresponds to a respective electrostatic charge variation indicative of the blink.

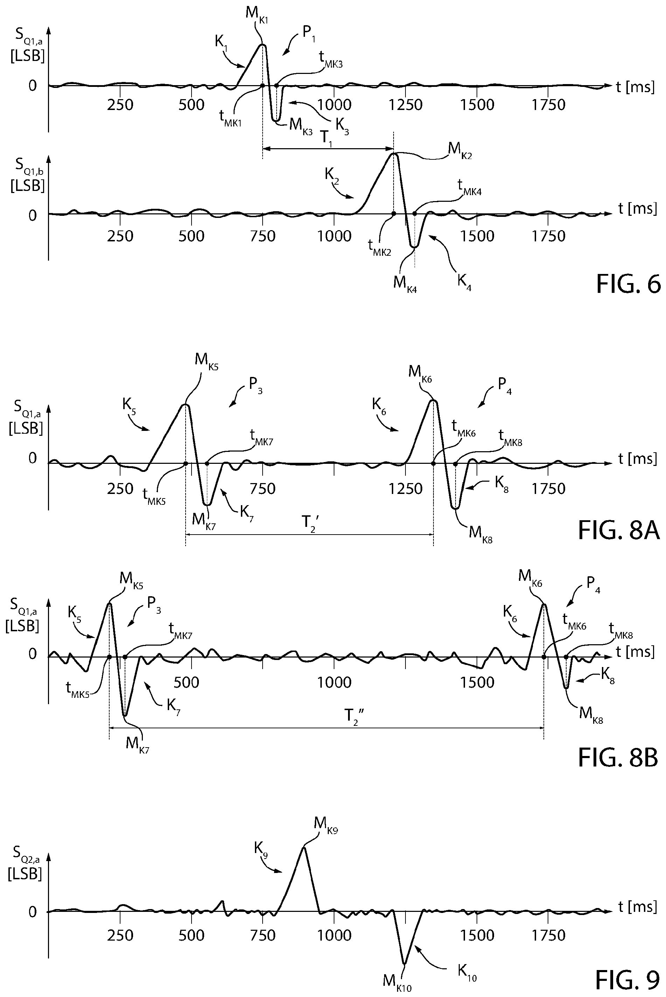

In greater detail, it has been verified that these movements of the eyes 30 in the presence of blinks generate, in each first electrostatic charge variation signal S_{Q,1} and in succession to each other in a blink period with a duration of less than about 50 ms, two respective peaks having opposite sign with respect to a baseline of this signal. In particular, by exemplarily considering a zero baseline, it is possible to have a first positive peak and a second negative peak or vice versa, as a function of the direction of movement of the eyes 30 and of the positions of the first and second electrodes 22a and 22b. These first and second consecutive peaks in the blink period define a blink scheme (or pattern) that is indicative of a blink (a voluntary or involuntary blink, as better discussed below). Examples of such blink patterns are provided in Figures 6-7C and better described below.

Instead, the movements of the eyes 30 in the absence of complete closure of the eyelids generate respective peaks, isolated from each other over time, in the second electrostatic charge variation signals S_{Q,2}. In particular, such peaks in the second electrostatic charge variation signals S_{Q,2} may be positive or negative (the latter are also referred to as valleys) with respect to respective baselines of the second electrostatic charge variation signals S_{Q,2} (e.g., here exemplarily considered to be equal to 0), as a function of the direction of movement of the eyes 30 and of the position of the third and the fourth electrodes 22c and 22d. An example of such peaks in the second electrostatic charge variation signals S_{Q,2} is provided in Figure 9 and is better described below.

In use, the main control unit 21 implements a detection method 50 of activity (i.e. of movement or state) of the eyes 30 of the user.

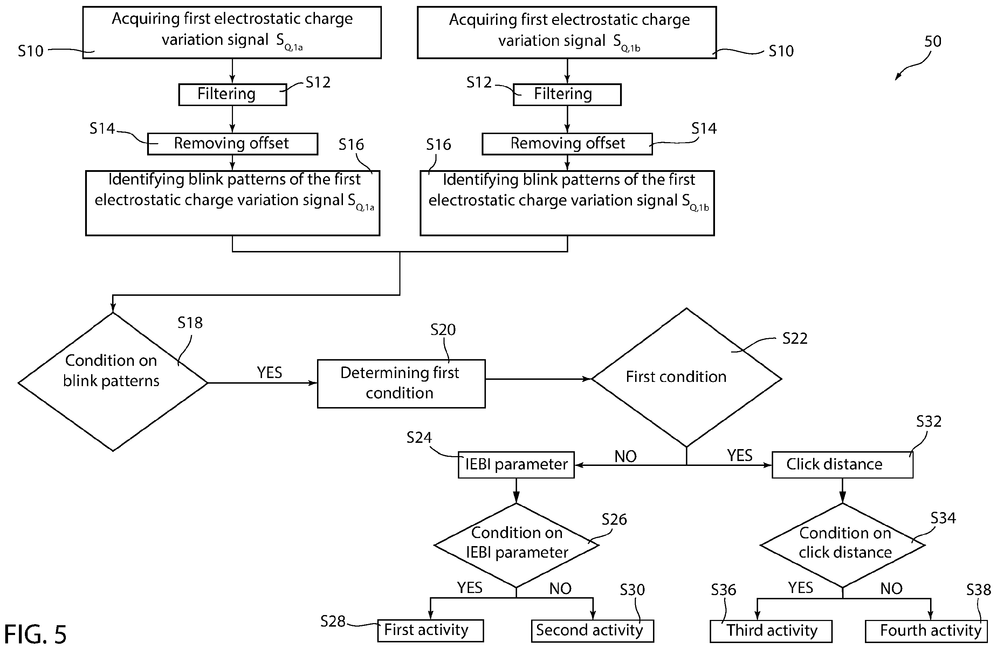

An embodiment of the detection method 50 is shown in Figure 5 and is now discussed.

The detection method 50 is performed iteratively, so as to update the information on the activity of the eyes 30 of the user in real time. For the sake of simplicity, an iteration of the detection method 50, also referred to as the current iteration, is described below.

At a step S10 of the detection method 50, the first electrostatic charge variation signals S_{Q,1} are acquired through the first electrostatic charge variation sensors 20a and 20b. Hereinafter, the first electrostatic charge variation signal S_{Q,1} acquired through the first left electrostatic charge variation sensor 20a is also indicated with the reference S_{Q,1a}, while the first electrostatic charge variation signal S_{Q,1} acquired through the first right electrostatic charge variation sensor 20b is also indicated with the reference S_{Q,1b}. In particular, the first electrostatic charge variation signals S_{Q,1a}, S_{Q,1b} are acquired through a scrolling buffer. In detail, at each iteration the first electrostatic charge variation signals S_{Q,1a}, S_{Q,1b} are acquired in a respective time window for example having a predefined duration equal to a time window period (e.g. equal to a few thousand ms, e.g. comprised between about 900 ms and about 2500 ms and e.g. equal to 1800 ms) greater than the blink period; in other words, at each iteration the first electrostatic charge variation signals S_{Q,1a}, S_{Q,1b} with a time duration equal to the time window period are considered. At each iteration, the oldest sample of each first electrostatic charge variation signal S_{Q,1a}, S_{Q,1b} is deleted and a new sample of each first electrostatic charge variation signal S_{Q,1a}, S_{Q,1b} is stored in the buffer.

In greater detail, at the current iteration the detection signals S_{R} of the first and second electrodes 22a and 22b of the first electrostatic charge variation sensors 20a and 20b are generated and the respective first electrostatic charge variation signals S_{Q,1a}, S_{Q,1b} are calculated as a function of the detection signals S_{R} (in particular, each first electrostatic charge variation signal S_{Q,1a}, S_{Q,1b} is equal to, or proportional to, the difference between the detection signals S_{R} of the first and the second electrodes 22a and 22b of the respective first electrostatic charge variation sensor 20a, 20b).

At a step S12, optional and immediately consecutive to step S10, the first electrostatic charge variation signals S_{Q,1a} and S_{Q,1b} are filtered to remove the contribution of the alternating electric current possibly present in the environment surrounding the glasses 10. In fact, if any, the alternating electric current generates respective electrostatic charge variations in the environment, which may be detected by the first electrostatic charge variation sensors 20a and 20b generating a respective peak, in the frequency domain, in the first electrostatic charge variation signals S_{Q,1a}, S_{Q,1b} at the frequency of the alternating electric current (i.e. 50 Hz or 60 Hz depending on the country one is in). In particular, the performed filtering may be of low-pass type with a cut-off frequency lower than a first threshold frequency (e.g., equal to about 25 Hz), or of band-pass type with a lower cut-off frequency greater than a second threshold frequency (lower than the first threshold frequency and for example equal to about 1 Hz) and with a higher cut-off frequency lower than the second threshold frequency and for example equal to 20 Hz, or of notch type with a lower cut-off frequency lower than the second threshold frequency and for example equal to 20 Hz, and with a higher cut-off frequency greater than a third threshold frequency (greater than the first threshold frequency and for example equal to about 80 Hz).

At a step S14, optional and immediately consecutive to step S12, the first electrostatic charge variation signals S_{Q,1a} and S_{Q,1b} are processed to remove an offset of the latter, and in detail to subtract a respective baseline of the latter (i.e. a reference value, not necessarily constant over time, around which the respective first electrostatic charge variation signal S_{Q,1a} and S_{Q,1b} develops; e.g., an average value) from each of them. In this manner, subsequently the variations of the first electrostatic charge variation signals S_{Q,1a} and S_{Q,1b} may be taken into account with respect to a zero baseline, and possible offset thereof are not to be considered.

At a step S16, immediately consecutive to step S14, the first electrostatic charge variation signals S_{Q,1a} and S_{Q,1b} are processed in a per se known manner to identify any peaks thereof (i.e. positive peaks or negative peaks, the latter also referred to as valleys). In detail, at step S16, if any, a number of peaks of the first electrostatic charge variation signals S_{Q,1a} and S_{Q,1b} and, for each peak, a respective maximum value and a respective time position (or instant) of this maximum value are identified. Greater details regarding the identification modes of these peaks may be found in the Italian patent document identified by the reference number 102021000012665, of the present Applicant. Alternatively, the portions of the first electrostatic charge variation signals S_{Q,1a} and S_{Q,1b} that have a greater value than a threshold value (e.g., a predefined value for example equal to about 200 LSB or a value equal to about 10% of the maximum value of the respective first electrostatic charge variation signal S_{Q,1a}, S_{Q,1b} in the considered time window) are considered.

At a step S18, immediately consecutive to step S16, it is determined whether a condition on the blink patterns of the first electrostatic charge variation signals S_{Q,1a} and S_{Q,1b} is verified. In particular, it is determined whether at least one blink pattern has been detected in at least one of the first electrostatic charge variation signals S_{Q,1a} and S_{Q,1b}. In other words, it is determined whether a sum of the number of blink patterns of the first electrostatic charge variation signals S_{Q,1a} and S_{Q,1b} is non-zero.

If the condition on the blink patterns is not verified (i.e. said sum is equal to zero), the current iteration of the detection method 50 ends and the method returns to step S10 to process the first electrostatic charge variation signals S_{Q,1a} and S_{Q,1a} in a subsequent time window.

If the condition on the blink patterns is verified (i.e. said sum is greater than zero), the detection method 50 proceeds to a step S20.

At step S20, immediately consecutive to step S18, it is verified whether the detected blink patterns of the first electrostatic charge variation signals S_{Q,1a} and S_{Q,1b} at the current iteration are indicative of a first condition of the eyes 30 of the user (i.e. whether a voluntary blink has occurred). In greater detail, for each pair of consecutive blink patterns of the first electrostatic charge variation signals S_{Q,1a} and S_{Q,1b}, the first condition is verified if these two blink patterns have a relative time distance greater than a first threshold period that is smaller than the time window period and greater than the blink period (for example it is equal to a few hundred ms, e.g. comprised between about 100 ms and about 800 ms and for example equal to 500 ms). For example, the relative time distance is calculated between reference points of the two blink patterns, similarly to what has been described hereinbelow with reference to the voluntary blink time distances. Therefore, when the first condition is verified, a voluntary blink has been detected at the current iteration.

A step S22, immediately consecutive to step S20, is a decision block that verifies whether the first condition has been detected or not at step S20.

In the present embodiment, if the first condition has not been verified (i.e. no voluntary blink has been detected) the detection method 50 proceeds to a step S24.

If the first condition has been verified (i.e. a voluntary blink has been detected), the detection method 50 proceeds to a step S32.

Step S24 refers to a second condition of the eyes 30 of the user (i.e. to an involuntary blink). In general, an involuntary blink has a shorter duration than a voluntary blink as it occurs in an involuntary manner, and involves both eyes simultaneously (unlike the voluntary blink that is performed with only one eye). In the present embodiment, the first and second conditions are alternative to each other, and therefore the second condition is detected when the first condition is not detected.

At step S24, an IEBI ("Inter-Eye blink interval") parameter, of a known type, is therefore calculated as a function of the first electrostatic charge variation signals S_{Q,1a} and S_{Q,2a}, and in particular as a function of the peaks of the first electrostatic charge variation signals S_{Q,1a} and S_{Q,1b}. The IEBI parameter is indicative of an involuntary blink frequency of the eyes 30 of the user, and in particular is indicative of an average, calculated over a predefined period (e.g., 1 minute), of the distances between involuntary blinks that are consecutive to each other over time.

In particular, Figure 6 shows the first electrostatic charge variation signals S_{Q,1a} and S_{Q,1b} in the time window of the current iteration, and two blink patterns of the first electrostatic charge variation signals S_{Q,1a} and S_{Q,1b} which define an involuntary blink. In detail, each involuntary blink is defined by a first blink pattern P₁ of one of the first electrostatic charge variation signals (here exemplarily S_{Q,1a}) and by a second blink pattern P₂ of the other first electrostatic charge variation signal (here exemplarily S_{Q,1b}), where the first and second blink patterns P₁ and P₂ are less distant from each other than the first threshold period. For example, the time positions t_{MK1} and t_{MK2} of the maximum values M_{K1} and M_{K2} of the first peaks (indicated in Figure 6 with the respective references K₁ and K₂) of the first and the second blink patterns P₁ and P₂ of the considered involuntary blink are distant from each other by a first time interval T₁ less than the first threshold period.

In greater detail, the IEBI parameter is indicative of the average, calculated in the predefined period, of the relative distances (not shown and hereinafter also referred to as involuntary blink time distances) between two consecutive involuntary blinks, and in detail between respective reference points of the two consecutive involuntary blinks. Each reference point may for example be a value such as the time position t_{MK1}, t_{MK2}, t_{MK3} or t_{MK4} of the maximum value M_{K1}, M_{K2}, M_{K3}, M_{K4} of the first (K₁ and K₂) or of the second (K₃ and K₄) peak of the first (P₁) or of the second (P₂) blink pattern of the involuntary blink. For example, the involuntary blink time distances used for the calculation of the IEBI parameter may be the distances between the time positions t_{MK1} of the maximum values M_{K1} of the first peaks K₁ of the first blink patterns P₁ of two consecutive involuntary blinks.

In particular, the IEBI parameter is updated at each iteration in which the second condition is verified. For example, at a first iteration (e.g., i=1) of the detection method 50, the IEBI parameter is set to a predefined value (e.g., equal to 5 s), and at each subsequent iteration in which the occurrence is detected of an involuntary blink (e.g., i=N) it is recalculated as a function of both the own value at the immediately preceding iteration (e.g., i=N-1) and the involuntary blink time distance calculated at the current iteration (e.g., i=N).

At a step S26, immediately consecutive to step S24, it is determined whether a condition on the IEBI parameter is verified or not, in order to determine an attention (or concentration) state of the user. In particular, it is verified whether the IEBI parameter is greater than an IEBI threshold value (e.g., comprised between about 3 s and about 12 s and for example equal to about 8 s) indicative of a threshold attention level of the user.

If the condition on the IEBI parameter is verified (i.e. the IEBI parameter is greater than the IEBI threshold value), a first activity of the eyes 30 of the user is detected (step S28). The first activity of the eyes 30 is indicative of a first attention state of the user which corresponds to a low (or lower) attention level of the user.

If the condition on the IEBI parameter is not verified (i.e. the IEBI parameter is lower than, or equal to, the IEBI threshold value), a second activity of the eyes 30 of the user is detected (step S30). The second activity of the eyes 30 is indicative of a second attention state of the user which corresponds to a high (or higher) attention level of the user.

As evident, the terms "high" and "low" mentioned in the present description with reference to the attention level are not to be understood in an absolute sense, but rather relatively to each other and in connection with the activity being performed (where the distinction is given by the IEBI threshold value).

At step S32 a relative distance (hereinafter also referred to as the voluntary blink time distance) between two detected and consecutive voluntary blinks of the first electrostatic charge variation signals S_{Q,1a} and S_{Q,1b} is calculated as a function of the first electrostatic charge variation signals S_{Q,1a} and S_{Q,1b} and in particular as a function of the peaks of the first electrostatic charge variation signals S_{Q,1a} and S_{Q,1b}.

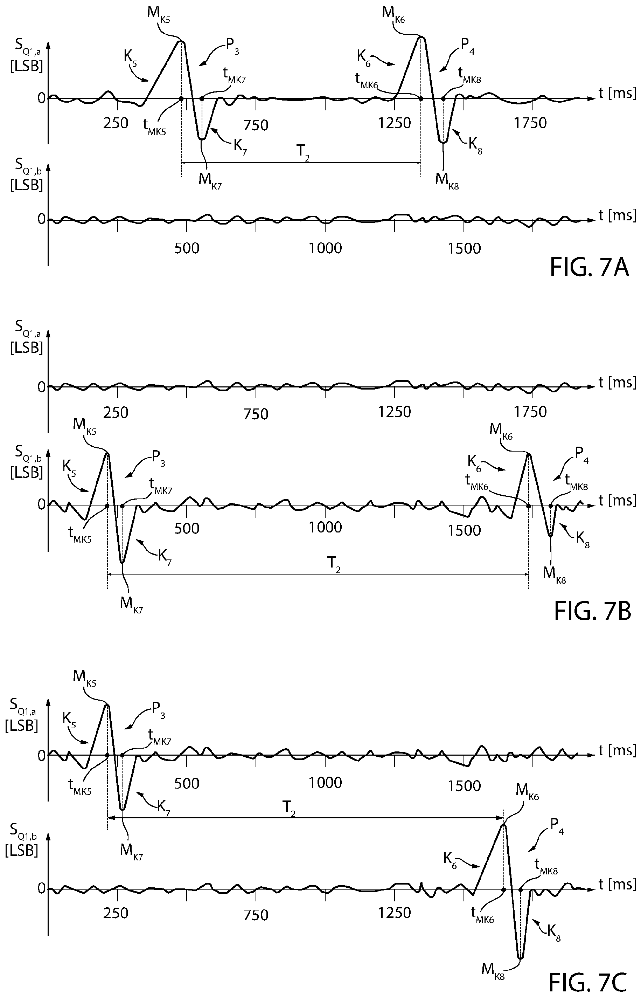

In particular, Figures 7A-7C each show the first electrostatic charge variation signals S_{Q,1a} and S_{Q,1b} in the current time window, and two blink patterns of the first electrostatic charge variation signals S_{Q,1a} and S_{Q,1b} which define two respective voluntary blinks. In detail, each voluntary blink is defined by a respective blink pattern (in Figures 7A-7C indicated for example by P₄) of one of the first electrostatic charge variation signals S_{Q,1a} and S_{Q,13}, where this blink pattern P₄ is distant from the immediately preceding blink pattern (in Figures 7A-7C indicated for example by P₃) more than the first threshold period. For example, the time positions t_{MK5} and t_{MK6} of the maximum values M_{K5} and M_{K6} of the first peaks K₅ and K₆ of the considered blink pattern P₄ and, respectively, of the immediately preceding blink pattern P₃ are distant from each other by a second time interval T₂ greater than the first threshold period. The blink pattern P₄ that defines the last considered voluntary blink may be part of the same first electrostatic charge variation signal of the previous blink pattern P₃ (S_{Q,1a} in Figure 7A and S_{Q,1b} in Figure 7B) or may be part of a first electrostatic charge variation signal (in Figure 7C, exemplarily S_{Q,1b}) different from that of the previous blink pattern P₃.

In greater detail, the voluntary blink time distance is defined between respective reference points of the last two detected voluntary blinks (P₃ and P₄ in Figures 7A-7C). Each reference point may for example be a value such as the time position t_{MK5}, t_{MK6}, t_{MK7} or t_{MK8} of the maximum value M_{K5}, M_{K6}, M_{K7}, M_{K8} of the first (K₅ and K₆) or of the second (K₇ and K₈) peak of the two blink patterns P₃ and P₄ which define said last two consecutive voluntary blinks. For example, the voluntary blink time distance may coincide with the second time interval T₂.

At a step S34, immediately consecutive to step S32, it is determined whether a condition on the voluntary blink time distance is verified or not, in order to determine a voluntary blink mode of the eyes 30 of the user. In particular, it is verified whether the voluntary blink time distance is less than a voluntary blink threshold distance (greater than the first threshold period, e.g. comprised between about 200 ms and about 1 s and for example equal to about 700 ms) indicative of a threshold level which identifies a double voluntary blink (better described hereinbelow).

If the condition on the voluntary blink time distance is verified (i.e. the voluntary blink time distance is less than the voluntary blink threshold distance), a third activity of the eyes 30 of the user is determined (step S36). The third activity is indicative of said double voluntary blink which corresponds to two voluntary blinks close to each other over time, as shown in Figure 8A with exemplary reference to the first electrostatic charge variation signal S_{Q,1a}. In other words, the double voluntary blink is made by two blink patterns between which the respective voluntary blink time distance (indicated in Figure 8A with the reference number T₂') is less than the voluntary blink threshold distance (also referred to as the second threshold period).

If the condition on the voluntary blink time distance is not verified (i.e. the voluntary blink time distance is greater than, or equal to, the voluntary blink threshold distance), a fourth activity of the eyes 30 of the user is determined (step S38). The fourth activity is indicative of a single voluntary blink, i.e. of a voluntary blink isolated over time, as shown in Figure 8B with exemplary reference to the first electrostatic charge variation signal S_{Q,1a}. In greater detail, the single voluntary blink is made by a blink pattern that has a voluntary blink time distance (indicated in Figure 8B with the reference number T₂"), with respect to the blink pattern of the immediately preceding voluntary blink, which is greater than the second threshold period.

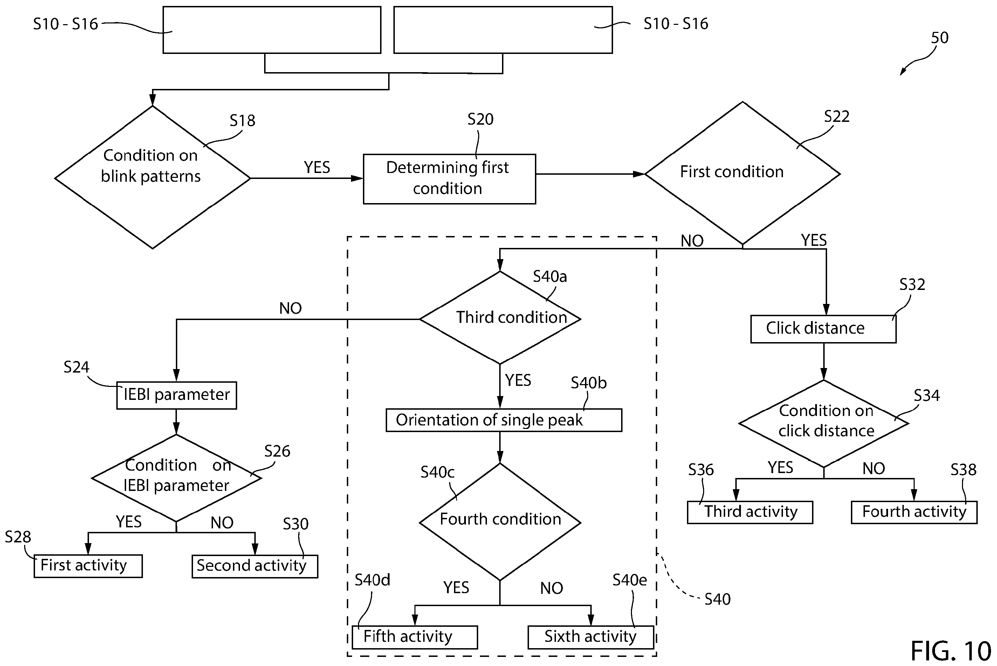

According to an embodiment of the detection method 50, shown in Figure 10, a plurality of further steps (indicated as a whole with the reference S40) are further comprised in the detection method 50 between step S22 and step S24.

Furthermore, in the present embodiment steps S10-S16 previously described are performed not only for the first electrostatic charge variation signals S_{Q,1a} and S_{Q,1b} but also for the second electrostatic charge variation signals S_{Q,2a} and S_{Q,2b} acquired through the third and fourth electrodes 22c and 22d of the second electrostatic charge variation sensors 20c and 20d. In particular, step S16 is performed for the second electrostatic charge variation signals S_{Q,2a} and S_{Q,2b} in order to detect possible peaks (positive or negative) thereof, similarly to what has been previously described for the detection of blink patterns in the first electrostatic charge variation signals S_{Q,1a} and S_{Q,1b}. In particular, Figure 9 shows an example of second electrostatic charge variation signal (here exemplarily S_{Q,2a}), wherein two peaks K₉ and K₁₀ are exemplarily shown, isolated from each other over time and which do not define any blink pattern. For example, the peak K₉ is positive (i.e. the respective maximum value M_{K9} is greater than 0, i.e. than the baseline of the second electrostatic charge variation signal S_{Q,2a}) and the peak K₁₀ is negative (i.e. the respective maximum value MK₁₀ is lower than 0, i.e. than the baseline of the second electrostatic charge variation signal S_{Q,2a}).

At a step S40a, consecutive to step S22 and interposed between step S22 and step S24, it is verified whether the second electrostatic charge variation signals S_{Q,2a} and S_{Q,2b} acquired at the current iteration through the third and fourth electrodes 22c and 22d are indicative of a third condition of the eyes 30 of the user. In greater detail, the third condition is indicative of a movement of the eyeballs of the eyes 30 in the orbital cavity in the absence of complete closure of the eyelids; in other words, the third condition is correlated to a rotation of the eyeballs which does not require the closure of the eyelids (i.e. the mutual contact of the upper eyelid and of the lower eyelid of each eye 30). The third condition is verified if the presence of at least one single peak is identified in each second electrostatic charge variation signal S_{Q,2a}, S_{Q,2b}. This single peak is a peak isolated over time with respect to the other peaks of the second electrostatic charge variation signal S_{Q,2a}, S_{Q,2b} and which is not part of any blink pattern (e.g., K₉ and K₁₀). In particular, the single peak of the second electrostatic charge variation signal S_{Q,2a}, S_{Q,2b} is a peak that has no counterpart in the first electrostatic charge variation signals S_{Q,1a}, S_{Q,1b} (i.e. the first electrostatic charge variation signals S_{Q,1a}, S_{Q,1b} have no peak at the time position of the single peak of the second electrostatic charge variation signal S_{Q,2a}, S_{Q,2b}). For example, each single peak has a time distance from the closest peak (e.g., measured between their maximum values) that is greater than a single peak threshold time distance (e.g., which is greater than the relative time distance of two peaks forming a blink pattern, such as the blink period).

If the third condition has not been verified (i.e. no movement of the eye 30 has been detected in the absence of complete closure of the eyelids), the detection method 50 proceeds to step S24 previously described as it is detected that the second condition is verified (i.e. an involuntary blink has been detected).

If the third condition has been verified (i.e. a movement of the eyes 30 has been detected in the absence of complete closure of the eyelids), the detection method 50 proceeds to a step S40b. Optionally, the method may also proceed simultaneously with both step S24 and step S40b.

At step S40b, consecutive to step S40a, the orientation is detected with respect to the baseline of the respective second electrostatic charge variation signal S_{Q,2a}, S_{Q,2b}, of the one or more single peaks detected in the second electrostatic charge variation signals S_{Q,2a}, S_{Q,2b} which identify respective movements of the eyeballs of the eyes 30 in the absence of complete closure of the eyelids. In detail, exemplarily considering only one second electrostatic charge variation signal S_{Q,2a}, S_{Q,2b} and only one single peak of the latter, at step S40b it is verified whether the single peak is positive (i.e. whether the respective maximum value is greater than the baseline, here considered zero in view of step S14) or negative (i.e. whether the respective maximum value is lower than the baseline, here considered zero in view of step S14).

A step S40c, consecutive to step S40b, is a decision step wherein it is determined whether, for each detected single peak of the second electrostatic charge variation signals S_{Q,2a}, S_{Q,2b}, a fourth condition which exemplarily corresponds to a predefined orientation of said single peak (e.g., positive orientation) is verified.

If the fourth condition has been verified (i.e. the single peak is positive), the detection method 50 proceeds to a step S40d.

If the fourth condition has not been verified (i.e. the single peak is negative), the detection method 50 proceeds to a step S40e.

If the fourth condition is verified, a fifth activity of the eyes 30 of the user is determined (step S40d). The fifth activity is indicative of a first movement of the eyes 30. For example, the first movement corresponds to a movement of the cornea 30a from right to left relative to the user's point of view, and/or to a movement of the cornea 30a from the third electrode 22c to the fourth electrode 22d of the second left electrostatic charge variation sensor 20c; nevertheless, the orientation of the first movement obviously depends on the position of the third and fourth electrodes 22c and 22d with respect to the eye 30, and therefore may also correspond, for example, to a movement of the cornea 30a from left to right.

If the fourth condition is not verified, a sixth activity of the eyes 30 of the user is determined (step S40e). The sixth activity is indicative of a second movement of the eyes 30. In particular, the second movement is opposite to the first movement; in other words it is performed in the opposite direction to the first movement. For example, the second movement corresponds to a movement of the cornea 30a from left to right, and/or to a movement of the cornea 30a from the fourth electrode 22d to the third electrode 22c; nevertheless, the orientation of the second movement obviously depends on the position of the third and fourth electrodes 22c and 22d with respect to the eye 30, and therefore may also correspond, for example, to a movement of the cornea 30a from right to left.

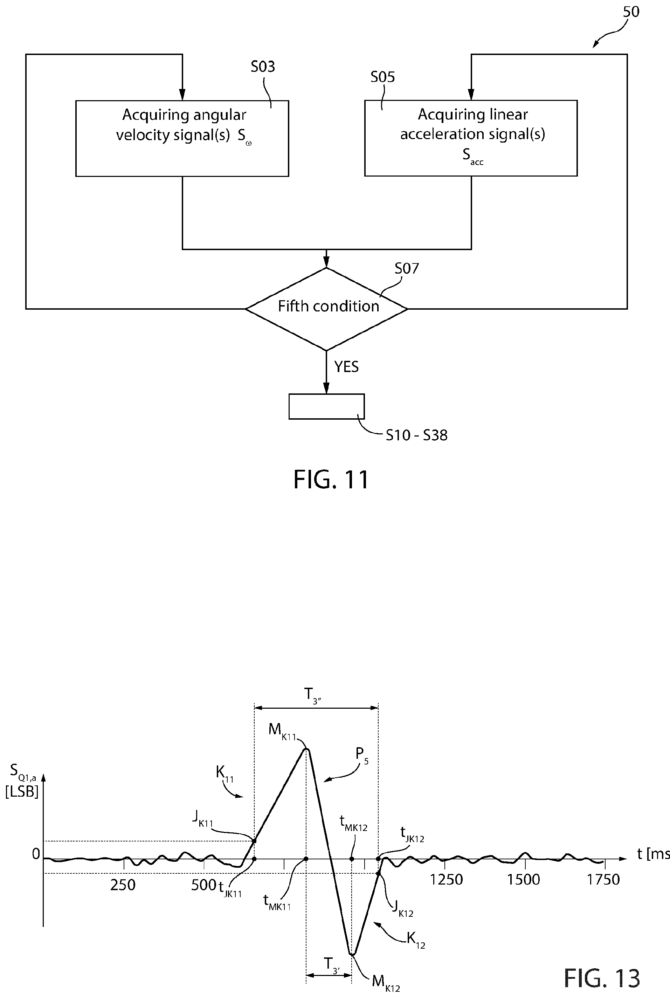

Figure 11 shows a further embodiment of the detection method 50.

In the present embodiment, the detection method 50 comprises further steps S03-S07 preceding step S10 and the glasses 10 further comprise one or more accelerometers (not shown) and one or more gyroscopes (not shown). Hereinafter, exemplary reference is made to the case of an accelerometer and a gyroscope, although a greater number of accelerometers and/or gyroscopes may similarly be considered. In particular, the accelerometer and the gyroscope are fixed to the glasses 10 (for example to the support portions 12a and/or 12b) and are configured to detect possible movements of the user's head (e.g., lateral rotations and forward or backward bending of the head, with respect to the torso).

At a step S03, the main control unit 21 acquires, through the gyroscope, one or more angular velocity signals S_{ω} indicative of respective angular velocities measured by the gyroscope. These one or more angular velocities are generated by movements of the user's head, for example with respect to the user's torso.

At a step S05, for example performed simultaneously with step S03, the main control unit 21 acquires, through the accelerometer, one or more linear acceleration signals S_{acc} indicative of respective linear accelerations measured by the accelerometer. These one or more linear accelerations are generated by movements of the user's head, for example with respect to the torso.

At a step S07, it is determined whether a fifth condition is verified, as a function of the angular velocity signals S_{ω} and of the linear acceleration signals S_{acc}. The fifth condition is determined when no movements of the user's head are detected. The determination of the movements of the head from the angular velocity signals S_{ω} and the linear acceleration signals S_{acc} is performed in a per se known manner (e.g., through machine learning techniques) and therefore not described in detail herein; nevertheless, details on this aspect may be found for example in the article "Absolute Orientation for Head-Tracking Using Gyroscope, Accelerometer, and Camera", Fisher, EE 267 - Virtual Reality - Stanford University - 2018.

If the fifth condition is not verified (i.e. movements of the head are detected) the detection method 50 ends and steps S10-S38 are not performed (e.g., the method returns to step S03).

If the fifth condition is verified (i.e. no movements of the head, which is immobile, are detected), the detection method 50 proceeds to step S10 and then the steps shown in Figure 5 or 10 are performed.

According to an embodiment not shown, the detection method 50 of Figures 5, 10 and 11 further comprises a step of controlling, as a function of the detected activity of the eyes 30, one or more functionalities of the same glasses 10 or of an apparatus (not shown) external to the glasses 10 and operatively coupled thereto (e.g., a smart TV or a smart household appliance). For purely illustrative and non-limiting purposes, the functionalities of the apparatus may comprise providing a sound alarm or activating a standby mode of the apparatus when the first activity (low attention level of the user) is detected, opening a folder or a document or selecting an option of the apparatus through the third and fourth activities (single and double voluntary blink), and scrolling through the text of a document through the fifth and sixth activities (movements of the eyes 30 without eyelid blinking).

From an examination of the characteristics of the invention made according to the present invention, the advantages that it affords are evident.

The glasses 10 and the detection method 50 previously described allow, owing to the proximity of the electrodes 22a-22d to the eyes 30, the movements of the eyes 30 to be detected with high accuracy. In particular, eye activities may be detected indicative of the attention level of the user, of single and double voluntary blinks and of movements of the eye 30 in the absence of blink.

The possibility, owing to the accelerometer and to the gyroscope, to perform the detection method 50 only in the absence of movements of the user's head avoids incorrect detections of movement of the eyes, actually due to movements of the head. Furthermore, the fact that the electrodes 22a-22d are carried by the glasses 10 and are not in contact with the skin of the user's face prevents detection errors due to the slipping of the same on the skin (e.g., when the latter is humid or sweaty).

The detection method 50 requires reduced computational resources to be implemented, and therefore minimizes the electrical consumption required.

Finally, it is clear that modifications and variations may be made to the invention described and illustrated herein without thereby departing from the scope of the present invention, as defined in the attached claims. For example, the embodiments described may be combined with each other to obtain further solutions.

Steps S24-S38 (as well as steps S40a-S40e if any) may be optional. In general, if the first condition is not determined at step S22, an involuntary blink is detected, and if the first condition is determined at step S22, a voluntary blink is detected. For example, if the first condition is not determined at step S22, a blink signal (not shown) is generated which assumes a first value (e.g., 0) indicative of the detection of an involuntary blink; if, on the other hand, the first condition is determined at step S22, the blink signal is generated with a second value (e.g., 1) indicative of the detection of a voluntary blink. For example, the blink signal may be used to control the one or more functionalities of the glasses 10 or of the apparatus, similarly to what has been previously described. Following the generation of the blink signal, steps S24-S38 (as well as steps S40a-S40e if any) may optionally be performed as previously described.

The glasses 10 may also comprise a single control unit coupled to the electrodes 22a-22d. For purely illustrative purposes, this control unit may comprise the main control unit 21 and the sensor control units 15 or it may be the main control unit 21 and the sensor control units 15 may be absent; in these cases, the actions previously described with reference to the sensor control units 15 of the charge variation sensors 20a, 20b are performed by this control unit.

Although the case in which the detection method 50 is performed by processing time windows superimposed on each other (scrolling buffer) has been previously described, the previous description applies in a similar manner also to the case of time windows that are consecutive and not superimposed on each other.

Furthermore, the number of electrostatic charge variation sensors may be greater than what has been previously considered. In particular, the glasses may comprise one or more third electrostatic charge variation sensors (not shown and similar to the second electrostatic charge variation sensors 20c and 20d), each of these comprising a respective pair of electrodes (not shown, for example a fifth and a sixth electrode) for an improved detection of the fifth and sixth activities. For example, the fifth and sixth electrodes are spaced from the electrodes 22a-22d, are radially internal with respect to the first and second electrodes 22a, 22b with respect to the center of the respective lens 14a, 14b, are distant from each other by the second mutual distance D₂ and are fixed to the respective lens 14a, 14b so as to face the eye 30 of the user when the latter wears the glasses 10. In particular, the fifth and sixth electrodes are angularly equi-spaced with respect to the third and fourth electrodes 22c and 22d with respect to the center of the respective lens 14a, 14b. For example, the fifth and sixth electrodes are aligned with each other along a second axis (not shown) orthogonal to the first axis, such that they are arranged as a cross with respect to the third and fourth electrodes 22c, 22d where the center of this cross corresponds to the center of the respective lens 14a, 14b (and therefore to a center of the eye 10, e.g. at the position of the pupil when the user's gaze is oriented along a direction orthogonal to the face). In this manner the movements of the eyes 30 in the absence of blink may be detected in a more efficient and accurate manner. Similarly to what has already been described for the third and fourth electrodes 20c and 20d, for each third electrostatic charge variation sensor the respective sensor control unit 15 is configured to process respective detection signals S_{R} acquired through the fifth and sixth electrodes and to generate a respective further second electrostatic charge variation signal indicative of a difference between the detection signals S_{R} acquired through the fifth and sixth electrodes, and therefore indicative of a difference between the electrostatic charge variations detected through the fifth and sixth electrodes.

Furthermore, the glasses 10 may comprise only one first electrostatic charge variation sensor (hereinafter exemplarily considered to be the first left electrostatic charge variation sensor 20a). In this case, the detected information refers only to one of the eyes 30 of the user (i.e. to the eye 30 having the first left electrostatic charge variation sensor 20a facing thereto). Nevertheless, the information obtained regarding the involuntary activity of one eye (e.g., involuntary blink and movements of the eyes in the absence of complete closure of the eyelids) may similarly be considered for the other eye as well, as the involuntary movements of the eyes (both involuntary blinks and movements of the eyes in the absence of blink) are generally substantially synchronous and dual. This consideration does not apply instead to voluntary movements of the eyes (e.g., voluntary blinks) that are deliberately performed by the user with only one eye.

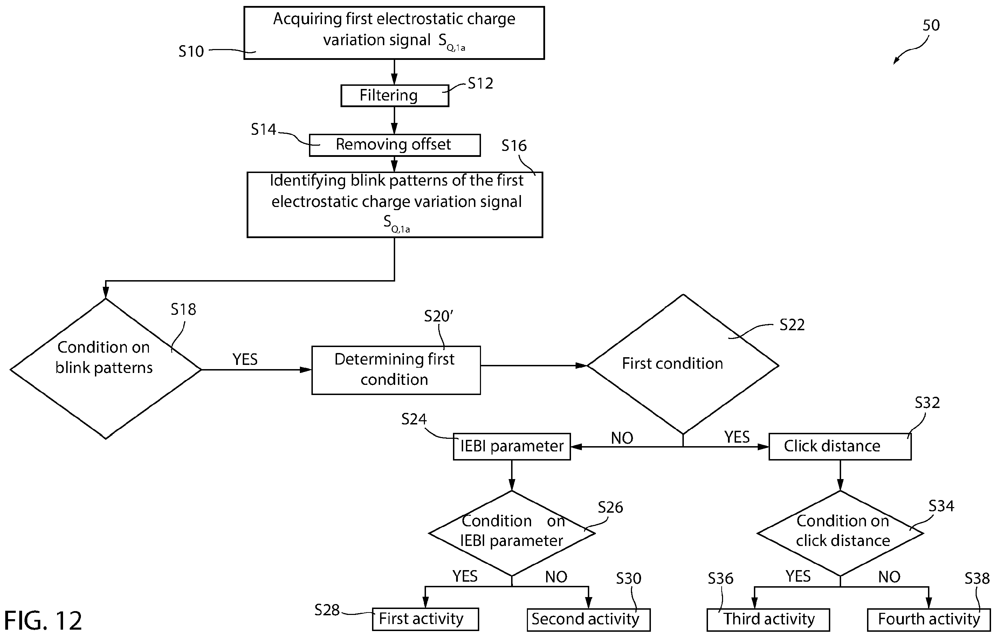

One embodiment of the detection method 50 corresponding to the case in which only the first left electrostatic charge variation sensor 20a is present is shown in Figure 12. In particular, when only the first left electrostatic charge variation sensor 20a is present the detection method 50 is similar to that previously described (in Figure 12 reference is made to the steps shown in Figure 5, although it is clear that what has been said also applies to the embodiments of Figures 10 and 11).

However, unlike the previously discussed embodiments, in Figure 12 steps S10-S16 are performed only for the first electrostatic charge variation signal S_{Q,1a}.

Furthermore, the determination of the first condition (previously step S20, replaced in Figure 12 with the new reference S20') occurs as a function of the sole first electrostatic charge variation signal S_{Q,1a}. In greater detail, at the current iteration, the first condition is verified (i.e. a voluntary blink has been detected) if a blink pattern of the first electrostatic charge variation signal S_{Q,1a}, which has a greater time duration than a voluntary blink threshold period (for example comprised between about 10 ms and about 40 ms, and for example equal to about 20 ms), is present. For example, Figure 13 shows the blink pattern (indicated with the reference P₅) which has the first peak K₁₁ with maximum value M_{K11} at the time position t_{MK11}, and the second peak K₁₂ with maximum value M_{K12} at the time position t_{MK12}. For purely illustrative and non-limiting purposes, the time duration may be a quantity such as the distance (indicated in Figure 13 with the reference T₃') between the time positions t_{MK11} and t_{MK12} of the maximum values M_{K11} and M_{K12} of the first and the second peaks K₁₁ and K₁₂ of the blink pattern P₅, or the distance (indicated in Figure 13 with the reference T₃") between the time positions t_{JK11} and t_{JK12} of values of interest J_{K11} and J_{K12} of the first and the second peaks K₁₁ and K₁₂ of the blink pattern P₅. For example, the values of interest J_{K11} and J_{K12} are points of the first electrostatic charge variation signal S_{Q,1a} which have a predefined value (e.g., 10% of the maximum value M_{K11}, M_{K12} of the respective peak K₁₁, K₁₂) and whose time positions t_{JK11}, t_{JK12} are preceding and, respectively, following the time positions t_{MK11}, t_{MK12} of the maximum values M_{K11}, M_{K12} of the respective peaks K₁₁, K₁₂. As a result, the first condition is verified (i.e. a voluntary blink has been detected) when the time duration T₃', T₃" of the blink pattern P₅ is greater than the voluntary blink threshold period.

Furthermore, in this embodiment at step S24 the IEBI parameter is determined as the average, over the predefined period, of the involuntary blink time distances, each involuntary blink time distance being calculated between two consecutive blink patterns of the first electrostatic charge variation signal S_{Q,1a}, each blink pattern defining a respective involuntary blink.

## Claims

1. A detection method (50) of an activity of a first eye (30) of a user of a pair of glasses (10) comprising a first electrostatic charge variation sensor (20a) including a first (22a) and a second (22b) electrode spaced from each other, facing the first eye (30) of the user and configured to detect respective electrostatic charge variations generated by a blink of the first eye (30), the glasses further comprising a control unit (21, 15) coupled to the first (22a) and second (22b) electrodes of the first electrostatic charge variation sensor (20a),
the detection method comprising the steps of:
- acquiring (S10-S14), by the control unit (21, 15) and through the first (22a) and second (22b) electrodes of the first electrostatic charge variation sensor (20a), a first electrostatic charge variation signal (S_{Q,1a}) indicative of a difference between the electrostatic charge variations detected by the first (22a) and the second (22b) electrodes of the first electrostatic charge variation sensor (20a);
- verifying (S16, S18), by the control unit (21, 15), the presence of one or more blink patterns in the first electrostatic charge variation signal (S_{Q,1a}), each blink pattern being indicative of a respective voluntary blink or of a respective involuntary blink of the first eye (30);
- if the first electrostatic charge variation signal (S_{Q,1a}) has said one or more blink patterns, for each blink pattern determining (S20, S22; S20', S22) by the control unit (21, 15) whether a first condition is verified, the first condition being verified if said blink pattern is indicative of a respective voluntary blink;
- if the first condition is not verified, detecting, by the control unit (21, 15), a respective involuntary blink;
- if the first condition is verified, detecting, by the control unit (21, 15), a respective voluntary blink,
wherein the glasses (10) further comprise a further first electrostatic charge variation sensor (20b) including a respective first (22a) and second (22b) electrode spaced from each other, facing a second eye (30) of the user and configured to detect respective electrostatic charge variations generated by a blink of the second eye (30), the control unit (21, 15) being further coupled to the first (22a) and second (22b) electrodes of the further first electrostatic charge variation sensor (20b),
the detection method further comprising the steps of:
- acquiring (S10-S14), by the control unit (21, 15) and through the first (22a) and second (22b) electrodes of the further first electrostatic charge variation sensor (20b), a further first electrostatic charge variation signal (S_{Q,1b}) indicative of a difference between the electrostatic charge variations detected by the first (22a) and the second (22b) electrodes of the further first electrostatic charge variation sensor (20b);
- verifying (S16, S18), by the control unit (21, 15), the presence of one or more respective blink patterns in the further first electrostatic charge variation signal (S_{Q,1b}),
wherein the first condition is verified if, for each pair of consecutive blink patterns of the first electrostatic charge variation signal (S_{Q,1b}) and/or of the further first electrostatic charge variation signal (S_{Q,1b}), a relative time distance between the blink patterns of said pair of blink patterns is greater than a first threshold period.

2. The detection method (50) according to claim 1, further comprising, if the first condition is not verified:
- determining (S24), by the control unit (21, 15) and as a function of said one or more blink patterns of the first electrostatic charge variation signal (S_{Q,1a}), an IEBI parameter indicative of an involuntary blink frequency;
- verifying (S26), by the control unit (21, 15), whether the IEBI parameter is greater than an IEBI threshold value;
- if the IEBI parameter is greater than the IEBI threshold value, detecting (S28) a first activity of the first eye (30), correlated to a lower attention level of the user;
- if the IEBI parameter is not greater than the IEBI threshold value, detecting (S30) a second activity of the first eye (30), correlated to a higher attention level of the user.

3. The detection method (50) according to claim 1 or 2, further comprising, if the first condition is verified:
- determining (S32), by the control unit (21, 15), as a function of said blink patterns of the first electrostatic charge variation signal (S_{Q,1a}) and for each pair of detected and consecutive voluntary blinks, a respective voluntary blink time distance between said voluntary blinks of the pair of voluntary blinks;
- verifying (S34), by the control unit (21, 15) and for each voluntary blink time distance, whether said voluntary blink time distance is less than a voluntary blink threshold distance;
- if the voluntary blink time distance is less than the voluntary blink threshold distance, detecting (S36) a third activity of the first eye (30) indicative of a double voluntary blink of the first eye (30);
- if the voluntary blink time distance is not less than the voluntary blink threshold distance, detecting (S38) a fourth activity of the first eye (30) indicative of a single voluntary blink of the first eye (30).

4. The detection method (50) according to claim 2 or claims 2 and 3, wherein the glasses (10) comprise a first lens (14a) facing the first eye (30),
wherein the glasses (10) comprise a second electrostatic charge variation sensor (20c) including a third (22c) and a fourth (22d) electrode spaced from each other, facing the first eye (30) of the user and configured to detect respective electrostatic charge variations generated by a movement of an eyeball of the first eye (30) in the absence of blink of the first eye (30), the first (22a) and second (22b) electrodes being radially external with respect to the third (22c) and fourth (22d) electrodes with respect to a center of the first lens (14a),
wherein the control unit (21, 15) is further coupled to the third (22c) and fourth (22d) electrodes of the second electrostatic charge variation sensor (20c),
wherein the detection method (50) further comprises:
- acquiring (S10-S14), from the control unit (21, 15) and through the third (22c) and fourth (22d) electrodes of the second electrostatic charge variation sensor (20c), a second electrostatic charge variation signal (S_{Q,2a}) indicative of a difference between the electrostatic charge variations detected by the third (22c) and fourth (22d) electrodes of the second electrostatic charge variation sensor (20c);
- if the first condition is verified, verifying (S40a), by the control unit (21, 15), the presence of one or more single peaks in the second electrostatic charge variation signal (S_{Q,2a}), each single peak being a respective peak of the second electrostatic charge variation signal (S_{Q,2a}), indicative of a respective movement of the eyeball of the first eye (30) in the absence of blink of the first eye (30);
- if said one or more single peaks are not present in the second electrostatic charge variation signal (S_{Q,2a}), determining (S24) the IEBI parameter;
- if said one or more single peaks are present in the second electrostatic charge variation signal (S_{Q,2a}), determining (S40b) the orientation of each of said single peaks with respect to a baseline of the second electrostatic charge variation signal (S_{Q,2a});
- for each of said one or more single peaks in the second electrostatic charge variation signal (S_{Q,2a}), verifying (S40c) whether the orientation is a predefined orientation;
- for each of said one or more single peaks in the second electrostatic charge variation signal (S_{Q,2a}), if the respective orientation is the predefined orientation, detecting (S40d) a fifth activity of the first eye (30), indicative of a first movement of the first eye (30);
- for each of said one or more single peaks in the second electrostatic charge variation signal (S_{Q,2a}), if the respective orientation is not the predefined orientation, detecting (S40e) a sixth activity of the first eye (30), indicative of a second movement of the first eye (30) opposite to the first movement.

5. The detection method (50) according to any of the preceding claims, wherein the step of verifying (S16, S18) the presence of said one or more blink patterns in the first electrostatic charge variation signal (S_{Q,1a}) comprises identifying, for each blink pattern, a first peak and a second peak in a blink period, the first peak and the second peak having sign opposite to each other with respect to a baseline of the second electrostatic charge variation signal (S_{Q,2a}) and forming said blink pattern.

6. The detection method (50) according to any of the preceding claims, wherein the step of detecting the involuntary blink comprises generating a blink signal with a first value indicative of the involuntary blink detection, and
wherein the step of detecting the voluntary blink comprises generating the blink signal with a second value indicative of the voluntary blink detection.

7. The detection method (50) according to any of the preceding claims, wherein the step of acquiring (S10-S14) the first electrostatic charge variation signal (S_{Q,1a}) comprises:
- acquiring respective detection signals (S_{R}) through the first (22a) and second (22b) electrodes, each detection signal (S_{R}) being indicative of the electrostatic charge variations on the respective first (22a) or second (22b) electrode;
- calculating a difference between said detection signals (S_{R}),
and comprises at least one of the following:
- filtering the difference between said detection signals (S_{R}); and
- removing an offset of the difference between said detection signals (S_{R}).

8. The detection method (50) according to any of the preceding claims, wherein the glasses (10) further comprise at least one accelerometer and at least one gyroscope coupled to the control unit (21, 15), and
wherein the detection method (50) further comprises:
- acquiring (S03), by the control unit (21, 15) and through the at least one gyroscope, one or more angular velocity signals (S_{ω}) indicative of respective angular velocities correlated to a movement of a user's head;
- acquiring (S05), by the control unit (21, 15) and through the at least one accelerometer, one or more linear acceleration signals (S_{acc}) indicative of respective linear accelerations correlated to the movement of the user's head;
- verifying (S07), by the control unit (21, 15) and as a function of the one or more angular velocity signals (S_{ω}) and of the one or more linear acceleration signals (S_{acc}), whether said movement of the user's head is present.

9. The detection method (50) according to any of the preceding claims when dependent on claim 2, wherein, if the first condition is not verified, the step of determining (S24) the IEBI parameter comprises calculating an involuntary blink time distance between two consecutive blink patterns of the first electrostatic charge variation signal (S_{Q,1a}), each blink pattern defining a respective involuntary blink.

10. The detection method (50) according to claim 2, wherein, if the first condition is not verified, the step of determining (S24) the IEBI parameter comprises calculating an involuntary blink time distance between two consecutive pairs of blink patterns of the first electrostatic charge variation signal (S_{Q,1a}) and of the further first electrostatic charge variation signal (S_{Q,1b}), each pair of blink patterns of the first electrostatic charge variation signal (S_{Q,1a}) and of the further first electrostatic charge variation signal (S_{Q,1b}) defining a respective involuntary blink.

11. The detection method (50) according to any of the preceding claims, further comprising the step of controlling, as a function of the detected activity of the first eye (30), one or more functionalities of the glasses (10) or of an apparatus operatively coupled to the glasses (10).

12. Glasses (10) wearable by a user and comprising:
a first electrostatic charge variation sensor (20a) including a first (22a) and a second (22b) electrode spaced from each other, facing a first eye (30) of the user and configured to detect respective electrostatic charge variations generated by a blink of the first eye (30), and
a control unit (21, 15) coupled to the first (22a) and second (22b) electrodes of the first electrostatic charge variation sensor (20a) and configured to:
- acquire (S10-S14), through the first (22a) and second (22b) electrodes of the first electrostatic charge variation sensor (20a), a first electrostatic charge variation signal (S_{Q,1a}) indicative of a difference between the electrostatic charge variations detected by the first (22a) and the second (22b) electrodes of the first electrostatic charge variation sensor (20a);
- verify (S16, S18) the presence of one or more blink patterns in the first electrostatic charge variation signal (S_{Q,1a}), each blink pattern being indicative of a respective voluntary blink or of a respective involuntary blink of the first eye (30);
- if the first electrostatic charge variation signal (S_{Q,1a}) has said one or more blink patterns, for each blink pattern determine (S20, S22; S20', S22) whether a first condition is verified, the first condition being verified if said blink pattern is indicative of a respective voluntary blink;
- if the first condition is not verified, detect a respective involuntary blink;
- if the first condition is verified, detect a respective voluntary blink,
wherein the glasses (10) further comprise a further first electrostatic charge variation sensor (20b) including a respective first (22a) and second (22b) electrode spaced from each other, facing a second eye (30) of the user and configured to detect respective electrostatic charge variations generated by a blink of the second eye (30),
the control unit (21, 15) being further coupled to the first (22a) and second (22b) electrodes of the further first electrostatic charge variation sensor (20b) and being further configured to:
- acquire (S10-S14), through the first (22a) and second (22b) electrodes of the further first electrostatic charge variation sensor (20b), a further first electrostatic charge variation signal (S_{Q,1b}) indicative of a difference between the electrostatic charge variations detected by the first (22a) and the second (22b) electrodes of the further first electrostatic charge variation sensor (20b); and
- verify (S16, S18) the presence of one or more respective blink patterns in the further first electrostatic charge variation signal (S_{Q,1b}),
wherein the first condition is verified if, for each pair of consecutive blink patterns of the first electrostatic charge variation signal (S_{Q,1b}) and/or of the further first electrostatic charge variation signal (S_{Q,1b}), a relative time distance between the blink patterns of said pair of blink patterns is greater than a first threshold period.

13. The glasses (10) according to claim 12, further comprising a second electrostatic charge variation sensor (20c) including a third (22c) and a fourth (22d) electrode spaced from each other and with respect to the first (22a) and second (22b) electrodes, facing the first eye (30) of the user and configured to detect respective electrostatic charge variations generated by a movement of an eyeball of the first eye (30) in the absence of blink of the first eye (30), the first (22a) and second (22b) electrodes of the first electrostatic charge variation sensor (20a) being radially external with respect to the third (22c) and fourth (22d) electrodes of the second electrostatic charge variation sensor (20c) with respect to a center of the first lens (14a), and
wherein the control unit (21, 15) is further coupled to the third (22c) and fourth (22d) electrodes of the second electrostatic charge variation sensor (20c).

14. The glasses (10) according to claim 13, further comprising a third electrostatic charge variation sensor including a fifth and a sixth electrode which are spaced from each other and with respect to the first (22a), the second (22b), the third (22c) and the fourth (22d) electrodes, face the first eye (30) of the user and are configured to detect respective electrostatic charge variations generated by the movement of the eyeball of the first eye (30) in the absence of blink of the first eye (30), the fifth and sixth electrodes being radially internal with respect to the first (22a) and second (22b) electrodes with respect to the center of the first lens (14a) and being angularly rotated with respect to the third (22c) and fourth (22d) electrodes around the center of the first lens (14a), and
wherein the control unit (21, 15) is further coupled to the fifth and sixth electrodes of the third electrostatic charge variation sensor.

15. A computer program product loadable into a control unit (21, 15) of the glasses according to claim 12,
said computer program being designed in such a way that, when run by the glasses according to claim 12 to execute the steps of the detection method (50) according to any of claims 1-11.

## Patentansprüche

1. Verfahren (50) zum Erkennen einer Aktivität eines ersten Auges (30) eines Benutzers einer Brille (10), umfassend einen ersten elektrostatischen Ladungsänderungssensor (20a), der eine erste (22a) und eine zweite (22b) Elektrode beinhaltet, die voneinander beabstandet sind, dem ersten Auge (30) des Benutzers zugewandt sind und konfiguriert sind, um jeweilige elektrostatische Ladungsänderungen zu erkennen, die durch einen Lidschlag des ersten Auges (30) erzeugt werden, wobei die Brille weiter eine Steuereinheit (21, 15) umfasst, die mit der ersten (22a) und zweiten (22b) Elektrode des ersten elektrostatischen Ladungsänderungssensors (20a) gekoppelt ist,
wobei das Verfahren zum Erkennen die Schritte umfasst zum:
- Erfassen (S10-S14), durch die Steuereinheit (21, 15) und durch die erste (22a) und zweite (22b) Elektrode des ersten elektrostatischen Ladungsänderungssensors (20a), eines ersten elektrostatischen Ladungsänderungssignals (S_{Q,1a}), das auf eine Differenz zwischen den von der ersten (22a) und der zweiten (22b) Elektrode des ersten elektrostatischen Ladungsänderungssensors (20a) erkannten elektrostatischen Ladungsänderungen hinweist;
- Überprüfen (S16, S18), durch die Steuereinheit (21, 15), des Vorhandenseins eines oder mehrerer Lidschlagmuster im ersten elektrostatischen Ladungsänderungssignal (S _{Q,1a}), wobei jedes Lidschlagmuster auf einen jeweiligen absichtlichen Lidschlag oder einen jeweiligen unabsichtlichen Lidschlag des ersten Auges (30) hinweist;
- wenn das erste elektrostatische Ladungsänderungssignal (S_{Q,1a}) das eine oder mehrere Lidschlagmuster aufweist, Bestimmen (S20, S22; S20', S22) für jedes Lidschlagmuster durch die Steuereinheit (21, 15), ob eine erste Bedingung überprüft ist, wobei die erste Bedingung überprüft ist, wenn das Lidschlagmuster auf einen jeweiligen absichtlichen Lidschlag hinweist;
- wenn die erste Bedingung nicht überprüft ist, Erkennen durch die Steuereinheit (21, 15) eines jeweiligen unabsichtlichen Lidschlags;
- wenn die erste Bedingung überprüft ist, Erkennen durch die Steuereinheit (21, 15) eines jeweiligen absichtlichen Lidschlags,
wobei die Brille (10) weiter einen weiteren ersten elektrostatischen Ladungsänderungssensor (20b) umfasst, der eine jeweilige erste (22a) und zweite (22b) Elektrode beinhaltet, die voneinander beabstandet sind, einem zweiten Auge (30) des Benutzers zugewandt sind und konfiguriert sind, um jeweilige elektrostatische Ladungsänderungen zu erkennen, die durch einen Lidschlag des zweiten Auges (30) erzeugt werden, wobei die Steuereinheit (21, 15) weiter mit der ersten (22a) und zweiten (22b) Elektrode des weiteren ersten elektrostatischen Ladungsänderungssensors (20b) gekoppelt ist,
wobei das Verfahren zum Erkennen weiter die Schritte umfasst zum:
- Erfassen (S10-S14), durch die Steuereinheit (21, 15) und durch die erste (22a) und zweite (22b) Elektrode des weiteren ersten elektrostatischen Ladungsänderungssensors (20b), eines weiteren ersten elektrostatischen Ladungsänderungssignals (S_{Q,1b}), das auf eine Differenz zwischen den von der ersten (22a) und der zweiten (22b) Elektrode des weiteren ersten elektrostatischen Ladungsänderungssensors (20b) erkannten elektrostatischen Ladungsänderungen hinweist;
- Überprüfen (S16, S18) durch die Steuereinheit (21, 15) des Vorhandenseins eines oder mehrerer jeweiliger Lidschlagmuster im weiteren ersten elektrostatischen Ladungsänderungssignal (S_{Q,1b}),
wobei die erste Bedingung überprüft ist, wenn für jedes Paar aufeinanderfolgender Lidschlagmuster des ersten elektrostatischen Ladungsänderungssignals (S_{Q,1b}) und/oder des weiteren ersten elektrostatischen Ladungsänderungssignals (S_{Q,1b}) ein relativer Zeitabstand zwischen den Lidschlagmustern des Paares von Lidschlagmustern größer ist als eine erste Schwellenperiode.

2. Verfahren (50) zum Erkennen nach Anspruch 1, weiter umfassend, wenn die erste Bedingung nicht überprüft ist:
- Bestimmen (S24) durch die Steuereinheit (21, 15) und in Abhängigkeit von dem einen oder den mehreren Lidschlagmustern des ersten elektrostatischen Ladungsänderungssignals (S_{Q,1a}) eines IEBI-Parameters, der auf eine unabsichtliche Lidschlagfrequenz hinweist;
- Überprüfen (S26) durch die Steuereinheit (21, 15), ob der IEBI-Parameter größer als ein IEBI-Schwellenwert ist;
- wenn der IEBI-Parameter größer als der IEBI-Schwellenwert ist, Erkennen (S28) einer ersten Aktivität des ersten Auges (30), die mit einem niedrigeren Aufmerksamkeitsniveau des Benutzers korreliert;
- wenn der IEBI-Parameter nicht größer als der IEBI-Schwellenwert ist, Erkennen (S30) einer zweiten Aktivität des ersten Auges (30), die mit einem höheren Aufmerksamkeitsniveau des Benutzers korreliert.

3. Verfahren (50) zum Erkennen nach Anspruch 1 oder 2, weiter umfassend, wenn die erste Bedingung überprüft ist:
- Bestimmen (S32) durch die Steuereinheit (21, 15) in Abhängigkeit von den Lidschlagmustern des ersten elektrostatischen Ladungsänderungssignals (S_{Q,1a}) und für jedes Paar erkannter und aufeinanderfolgender absichtlicher Lidschlagsignale eines jeweiligen absichtlichen Lidschlagzeitabstands zwischen den absichtlichen Lidschlagsignalen des Paares absichtlicher Lidschlagsignale;
- Überprüfen (S34) durch die Steuereinheit (21, 15) und für jeden absichtlichen Lidschlagzeitabstand, ob der absichtliche Lidschlagzeitabstand kleiner als ein absichtlicher Lidschlagschwellenabstand ist;
- wenn der absichtliche Lidschlagzeitabstand kleiner als der absichtliche Lidschlagschwellenabstand ist, Erkennen (S36) einer dritten Aktivität des ersten Auges (30), die auf einen doppelten absichtlichen Lidschlag des ersten Auges (30) hinweist;
- wenn der absichtliche Lidschlagzeitabstand nicht kleiner als der absichtliche Lidschlagschwellenabstand ist, Erkennen (S38) einer vierten Aktivität des ersten Auges (30), die auf einen einzelnen absichtlichen Lidschlag des ersten Auges (30) hinweist.

4. Verfahren (50) zum Erkennen nach Anspruch 2 oder den Ansprüchen 2 und 3, wobei die Brille (10) eine erste Linse (14a) umfasst, die dem ersten Auge (30) zugewandt ist,
wobei die Brille (10) einen zweiten elektrostatischen Ladungsänderungssensor (20c) umfasst, der eine dritte (22c) und eine vierte (22d) Elektrode beinhaltet, die voneinander beabstandet sind, dem ersten Auge (30) des Benutzers zugewandt sind und konfiguriert sind, um jeweilige elektrostatische Ladungsänderungen zu erkennen, die durch eine Bewegung eines Augapfels des ersten Auges (30) beim Nicht-Vorhandensein eines Lidschlags des ersten Auges (30) erzeugt werden, wobei die erste (22a) und zweite (22b) Elektrode in Bezug auf die dritte (22c) und vierte (22d) Elektrode in Bezug auf eine Mitte der ersten Linse (14a) radial außen sind,
wobei die Steuereinheit (21, 15) ferner mit der dritten (22c) und vierten (22d) Elektrode des zweiten elektrostatischen Ladungsänderungssensors (20c) gekoppelt ist,
wobei das Verfahren (50) zum Erkennen weiter umfasst:
- Erfassen (S10-S14), von der Steuereinheit (21, 15) und durch die dritte (22c) und vierte (22d) Elektrode des zweiten elektrostatischen Ladungsänderungssensors (20c), eines zweiten elektrostatischen Ladungsänderungssignals (S _{Q,2a} ), das auf eine Differenz zwischen den von der dritten (22c) und vierten (22d) Elektrode des zweiten elektrostatischen Ladungsänderungssensors (20c) erkannten elektrostatischen Ladungsänderungen hinweist;
- wenn die erste Bedingung überprüft ist, Überprüfen (S40a), durch die Steuereinheit (21, 15), des Vorhandenseins einer oder mehrerer Einzelspitzen im zweiten elektrostatischen Ladungsänderungssignal (S_{Q,2a}), wobei jede Einzelspitze eine jeweilige Spitze des zweiten elektrostatischen Ladungsänderungssignals (S_{Q,2a}) ist, die auf eine entsprechende Bewegung des Augapfels des ersten Auges (30) bei Nicht-Vorhandensein des Lidschlags des ersten Auges (30) hinweist;
- wenn die eine oder mehrere Einzelspitzen nicht im zweiten elektrostatischen Ladungsänderungssignal (S_{Q,2a}) vorhanden sind, Bestimmen (S24) des IEBI-Parameters;
- wenn die eine oder mehrere Einzelspitzen im zweiten elektrostatischen Ladungsänderungssignal (S_{Q,2a}) vorhanden sind, Bestimmen (S40b) der Ausrichtung jeder der Einzelspitzen in Bezug auf eine Basislinie des zweiten elektrostatischen Ladungsänderungssignals (S_{Q,2a});
- für jede der einen oder mehreren Einzelspitzen im zweiten elektrostatischen Ladungsänderungssignal (S_{Q,2a}), Überprüfen (S40c), ob die Ausrichtung eine vordefinierte Ausrichtung ist;
- für jede der einen oder mehreren Einzelspitzen im zweiten elektrostatischen Ladungsänderungssignal (S_{Q,2a}), wenn die jeweilige Ausrichtung die vordefinierte Ausrichtung ist, Erkennen (S40d) einer fünften Aktivität des ersten Auges (30), die auf eine erste Bewegung des ersten Auges (30) hinweist;
- für jede der einen oder mehreren Einzelspitzen im zweiten elektrostatischen Ladungsänderungssignal (S_{Q,2a}), wenn die jeweilige Ausrichtung nicht die vordefinierte Ausrichtung ist, Erkennen (S40e) einer sechsten Aktivität des ersten Auges (30), die auf eine der ersten Bewegung entgegengesetzte zweite Bewegung des ersten Auges (30) hinweist.

5. Verfahren (50) zum Erkennen nach einem der vorstehenden Ansprüche, wobei der Schritt zum Überprüfen (S16, S18) des Vorhandenseins des einen oder mehrerer Lidschlagmuster im ersten elektrostatischen Ladungsänderungssignal (S_{Q,1a}) Identifizieren für jedes Lidschlagmuster einer ersten Spitze und einer zweiten Spitze in einer Lidschlagperiode, wobei die erste Spitze und die zweite Spitze in Bezug auf eine Baseline des zweiten elektrostatischen Ladungsänderungssignals (S_{Q,2a}) entgegengesetzte Vorzeichen zueinander aufweisen und Bilden des Lidschlagmusters umfasst.

6. Verfahren (50) zum Erkennen nach einem der vorstehenden Ansprüche, wobei der Schritt des Erkennens des unabsichtlichen Lidschlags Erzeugen eines Lidschlagsignals mit einem ersten Wert umfasst, der auf die Erkennung des unabsichtlichen Lidschlags hinweist, und
wobei der Schritt des Erkennens des absichtlichen Lidschlags Erzeugen des Lidschlagsignals mit einem zweiten Wert umfasst, der auf die Erkennung des absichtlichen Lidschlags hinweist.

7. Verfahren (50) zum Erkennen nach einem der vorstehenden Ansprüche, wobei der Schritt des Erfassens (S10-S14) des ersten elektrostatischen Ladungsänderungssignals (S_{Q,1a}) umfasst:
- Erfassen von jeweiligen Erkennungssignalen (S_{R}) durch die erste (22a) und zweite (22b) Elektrode, wobei jedes Erkennungssignal (S_{R}) auf die elektrostatischen Ladungsänderungen an der jeweiligen ersten (22a) oder zweiten (22b) Elektrode hinweist;
- Berechnen einer Differenz zwischen den Erkennungssignalen (S_{R}),
und mindestens eines von Folgendem umfasst:
- Filtern der Differenz zwischen den Erkennungssignalen (S_{R}); und
- Entfernen eines Versatzes der Differenz zwischen den Erkennungssignalen (S_{R}).

8. Verfahren (50) zum Erkennen nach einem der vorstehenden Ansprüche, wobei die Brille (10) weiter mindestens einen Beschleunigungsmesser und mindestens ein mit der Steuereinheit (21, 15) gekoppeltes Gyroskop umfasst, und wobei das Verfahren (50) zum Erkennen weiter umfasst:
- Erfassen (S03), durch die Steuereinheit (21, 15) und durch das mindestens eine Gyroskop, eines oder mehrerer Winkelgeschwindigkeitssignale (Sω), die auf jeweilige Winkelgeschwindigkeiten hinweisen, die mit einer Bewegung des Kopfes eines Benutzers korrelieren;
- Erfassen (S05), durch die Steuereinheit (21, 15) und durch den mindestens einen Beschleunigungsmesser, eines oder mehrerer linearer Beschleunigungssignale (S_{acc}), die auf jeweilige lineare Beschleunigungen hinweisen, die mit der Bewegung des Kopfes des Benutzers korrelieren;
- Überprüfen (S07), durch die Steuereinheit (21, 15) und in Abhängigkeit von dem einen oder mehreren Winkelgeschwindigkeitssignalen (Sω) und dem einen oder mehreren linearen Beschleunigungssignalen (S_{acc}), ob die Bewegung des Kopfes des Benutzers vorhanden ist.

9. Verfahren (50) zum Erkennen nach einem der vorstehenden Ansprüche, wenn von Anspruch 2 abhängig, wobei, wenn die erste Bedingung nicht überprüft ist, der Schritt zum Bestimmen (S24) des IEBI-Parameters Berechnen eines unabsichtlichen Lidschlagzeitabstands zwischen zwei aufeinanderfolgenden Lidschlagmustern des ersten elektrostatischen Ladungsänderungssignals (S_{Q,1a}) umfasst, wobei jedes Lidschlagmuster einen jeweiligen unabsichtlichen Lidschlag definiert.

10. Verfahren (50) zum Erkennen nach Anspruch 2, wobei, wenn die erste Bedingung nicht überprüft ist, der Schritt zum Bestimmen (S24) des IEBI-Parameters Berechnen eines unabsichtlichen Lidschlagzeitabstands zwischen zwei aufeinanderfolgenden Lidschlagmusterpaaren des ersten elektrostatischen Ladungänderungssignals (S_{Q,1a}) und des weiteren ersten elektrostatischen Ladungänderungssignals (S _{Q,1b} ) umfasst, wobei jedes Lidschlagmusterpaar des ersten elektrostatischen Ladungänderungssignals (S_{Q,1a}) und des weiteren ersten elektrostatischen Ladungänderungssignals (S_{Q,1b}) einen jeweiligen unabsichtlichen Lidschlag definiert.

11. Verfahren (50) zum Erkennen nach einem der vorstehenden Ansprüche, weiter umfassend den Schritt zum Steuern, in Abhängigkeit von der erkannten Aktivität des ersten Auges (30), einer oder mehrerer Funktionalitäten der Brille (10) oder einer mit der Brille (10) wirkgekoppelten Einrichtung.

12. Brille (10), die von einem Benutzer tragbar ist und umfasst:
einen ersten elektrostatischen Ladungsänderungssensor (20a), der eine erste (22a) und eine zweite (22b) Elektrode beinhaltet, die voneinander beabstandet sind, einem ersten Auge (30) des Benutzers zugewandt sind und konfiguriert sind, um jeweilige elektrostatische Ladungsänderungen zu erkennen, die durch einen Lidschlag des ersten Auges (30) erzeugt werden, und
eine Steuereinheit (21, 15), die mit der ersten (22a) und zweiten (22b) Elektrode des ersten elektrostatischen Ladungsänderungssensors (20a) gekoppelt und konfiguriert ist, um:
- durch die erste (22a) und die zweite (22b) Elektrode des ersten elektrostatischen Ladungsänderungssensors (20a) ein erstes elektrostatisches Ladungsänderungssignal (S_{Q,1a}), das auf eine Differenz zwischen den von der ersten (22a) und der zweiten (22b) Elektrode des ersten elektrostatischen Ladungsänderungssensors (20a) erkannten elektrostatischen Ladungsänderungen hinweist, zu erfassen (S10-S14);
- das Vorhandensein eines oder mehrerer Lidschlagmuster im ersten elektrostatischen Ladungsänderungssignal (S _{Q,1a}) zu überprüfen (S16, S18), wobei jedes Lidschlagmuster auf einen jeweiligen absichtlichen Lidschlag oder einen jeweiligen unabsichtlichen Lidschlag des ersten Auges (30) hinweist;
- wenn das erste elektrostatische Ladungsänderungssignal (S_{Q,1a}) das eine oder mehrere Lidschlagmuster aufweist, für jedes Lidschlagmuster zu bestimmen (S20, S22; S20', S22), ob eine erste Bedingung überprüft ist, wobei die erste Bedingung überprüft ist, wenn das Lidschlagmuster auf einen jeweiligen absichtlichen Lidschlag hinweist;
- wenn die erste Bedingung nicht überprüft ist, einen jeweiligen unabsichtlichen Lidschlag zu erkennen;
- wenn die erste Bedingung überprüft ist, einen jeweiligen absichtlichen Lidschlag zu erkennen,
wobei die Brille (10) weiter einen weiteren ersten elektrostatischen Ladungsänderungssensor (20b) umfasst,
der eine jeweilige erste (22a) und zweite (22b) Elektrode beinhaltet, die voneinander beabstandet sind, einem zweiten Auge (30) des Benutzers zugewandt sind und konfiguriert sind, um jeweilige elektrostatische Ladungsänderungen zu erkennen, die durch einen Lidschlag des zweiten Auges (30) erzeugt werden,
wobei die Steuereinheit (21, 15) weiter mit der ersten (22a) und zweiten (22b) Elektrode des weiteren ersten elektrostatischen Ladungsänderungssensors (20b) gekoppelt und weiter konfiguriert ist, um:
- durch die erste (22a) und zweite (22b) Elektrode des weiteren ersten elektrostatischen Ladungänderungssensors (20b), ein weiteres erstes elektrostatisches Ladungsschwankungssignal (S_{Q,1b}), das auf eine Differenz zwischen den von der ersten (22a) und der zweiten (22b) Elektrode des weiteren ersten elektrostatischen Ladungsänderungssensors (20b) erkannten elektrostatischen Ladungsänderungen hinweist, zu erfassen (S10-S14); und
- das Vorhandensein eines oder mehrerer jeweiliger Lidschlagmuster im weiteren ersten elektrostatischen Ladungsänderungssignal (S_{Q,1b}) zu überprüfen (S16, S18),
wobei die erste Bedingung überprüft ist, wenn für jedes Paar aufeinanderfolgender Lidschlagmuster des ersten elektrostatischen Ladungsänderungssignals (S_{Q,1b}) und/oder des weiteren ersten elektrostatischen Ladungsänderungssignals (S_{Q,1b}) ein relativer Zeitabstand zwischen den Lidschlagmustern des Paares von Lidschlagmustern größer ist als eine erste Schwellenperiode.

13. Brille (10) nach Anspruch 12, weiter umfassend einen zweiten elektrostatischen Ladungsänderungssensor (20c), der eine dritte (22c) und eine vierte (22d) Elektrode umfasst, die voneinander und in Bezug auf die erste (22a) und zweite (22b) Elektrode beabstandet sind, dem ersten Auge (30) des Benutzers zugewandt sind und konfiguriert sind, um die jeweiligen elektrostatischen Ladungsänderungen zu erkennen, die durch eine Bewegung eines Augapfels des ersten Auges (30) bei Nicht-Vorhandensein eines Lidschlags des ersten Auges (30) erzeugt werden, wobei die erste (22a) und zweite (22b) Elektrode des ersten elektrostatischen Ladungsänderungssensors (20a) in Bezug auf die dritte (22c) und vierte (22d) Elektrode des zweiten elektrostatischen Ladungsänderungssensors (20c) in Bezug auf eine Mitte der ersten Linse (14a) radial außen sind, und
wobei die Steuereinheit (21, 15) weiter mit der dritten (22c) und vierten (22d) Elektrode des zweiten elektrostatischen Ladungsänderungssensors (20c) gekoppelt ist.

14. Brille (10) nach Anspruch 13, weiter umfassend einen dritten elektrostatischen Ladungsänderungssensor, der eine fünfte und eine sechste Elektrode beinhaltet, die voneinander und in Bezug auf die erste (22a), die zweite (22b), die dritte (22c) und die vierte (22d) Elektrode beabstandet sind, dem ersten Auge (30) des Benutzers zugewandt sind und konfiguriert sind, um jeweilige elektrostatische Ladungsänderungen zu erkennen, die durch eine Bewegung des Augapfels des ersten Auges (30) bei Nicht-Vorhandensein eines Lidschlags des ersten Auges (30) erzeugt werden, wobei die fünfte und sechste Elektrode in Bezug auf die erste (22a) und zweite (22b) Elektrode in Bezug auf die Mitte der ersten Linse (14a) radial innen sind und in Bezug auf die dritte (22c) und vierte (22d) Elektrode um die Mitte der ersten Linse (14a) winklig gedreht sind, und
wobei die Steuereinheit (21, 15) weiter mit der fünften und sechsten Elektrode des dritten elektrostatischen Ladungsänderungssensors gekoppelt ist.

15. Computerprogrammprodukt, das in eine Steuereinheit (21, 15) der Brille nach Anspruch 12 ladbar ist, wobei das Computerprogramm gestaltet ist, sodass, wenn es in der Brille nach Anspruch 12 läuft, die Schritte des Verfahrens (50) zum Erkennen nach einem der Ansprüche 1 bis 11 ausführt.

## Revendications

1. Procédé (50) de détection d'une activité d'un premier œil (30) d'un utilisateur d'une paire de lunettes (10) comprenant un premier capteur (20a) de variation de charge électrostatique comportant des première (22a) et deuxième (22b) électrodes espacées l'une de l'autre, orientées vers le premier œil (30) de l'utilisateur et configurées pour détecter des variations de charge électrostatique respectives générées par un clignement du premier œil (30), les lunettes comprenant en outre une unité (21, 15) de commande couplée aux première (22a) et deuxième (22b) électrodes du premier capteur (20a) de variation de charge électrostatique,
le procédé de commande comprenant les étapes suivantes :
- l'acquisition (S10-S14), par l'unité (21, 15) de commande et par le biais des première (22a) et deuxième (22b) électrodes du premier capteur (20a) de variation de charge électrostatique, d'un premier signal (S_{Q,1a}) de variation de charge électrostatique indiquant une différence entre les variations de charge électrostatique détectées par les première (22a) et deuxième (22b) électrodes du premier capteur (20a) de variation de charge électrostatique ;
- la vérification (S16, S18), par l'unité (21, 15) de commande, de la présence d'un ou de plusieurs schémas de clignement dans le premier signal (S_{Q,1a}) de variation de charge électrostatique , chaque schéma de clignement indiquant un clignement volontaire respectif ou un clignement involontaire respectif du premier œil (30) ;
- si le premier signal (S_{Q,1a}) de variation de charge électrostatique indique un ou plusieurs schémas de clignement, la détermination (S20, S22 ; S20', S22) pour chaque schéma de clignement par l'unité (21, 15) de commande si oui ou non une première condition est vérifiée, la première condition étant vérifiée si ledit schéma de clignement indique un clignement volontaire respectif ;
- si la première condition n'est pas vérifiée, la détection, par l'unité de commande (21, 15), d'un clignement involontaire respectif ;
- si la première condition est vérifiée, la détection, par l'unité (21, 15) de commande, d'un clignement volontaire respectif,
dans lequel les lunettes (10) comprennent en outre un premier capteur (20b) de variation de charge électrostatique supplémentaire comportant des première (22a) et deuxième (22b) électrodes respectives espacées l'une de l'autre, orientées vers un second œil (30) de l'utilisateur et configurées pour détecter des variations respectives de charge électrostatique générées par un clignement du second œil (30), l'unité (21, 15) de commande étant en outre couplée aux première (22a) et deuxième (22b) électrodes du premier capteur (20b) de variation de charge électrostatique supplémentaire,
le procédé de détection comprenant en outre les étapes suivantes :
- l'acquisition (S10-S14), par l'unité (21, 15) de commande et par le biais des première (22a) et deuxième (22b) électrodes du premier capteur (20b) de variation de charge électrostatique supplémentaire, d'un premier signal (S_{Q,1b}) de variation de charge électrostatique supplémentaire indiquant une différence entre les variations de charge électrostatique détectées par les première (22a) et deuxième (22b) électrodes du premier capteur (20b) de variation de charge électrostatique supplémentaire ;
- la vérification (S16, S18), par l'unité (21, 15) de commande, de la présence d'un ou de plusieurs schémas de clignement respectifs dans le premier signal (S_{Q,1b}) de variation de charge électrostatique supplémentaire,
dans lequel la première condition est vérifiée si, pour chaque paire de modèles de clignement consécutifs du premier signal (S_{Q,1b}) de variation de charge électrostatique et/ou du premier signal (S_{Q,1b}) de variation de charge électrostatique supplémentaire, une distance temporelle relative entre les modèles de clignement de ladite paire de modèles de clignement est supérieure à une première période seuil.

2. Procédé (50) de détection selon la revendication 1, comprenant en outre, si la première condition n'est pas vérifiée :
- la détermination (S24), par l'unité (21, 15) de commande et en fonction desdits un ou plusieurs schémas de clignement du premier signal (S_{Q,1a}) de variation de charge électrostatique, d'un paramètre IEBI indiquant une fréquence de clignement involontaire ;
- la vérification (S26), par l'unité (21, 15) de commande, si oui ou non le paramètre IEBI est supérieur à une valeur seuil IEBI ;
- si le paramètre IEBI est supérieur à la valeur seuil IEBI, la détection (S28) d'une première activité du premier œil (30), corrélée à un niveau d'attention inférieur de l'utilisateur ;
- si le paramètre IEBI n'est pas supérieur à la valeur seuil IEBI, la détection (S30) d'une deuxième activité du premier œil (30), corrélée à un niveau d'attention supérieur de l'utilisateur.

3. Procédé (50) de détection selon la revendication 1 ou 2, comprenant en outre, si la première condition est vérifiée :
- la détermination (S32), par l'unité (21, 15) de commande, en fonction desdits modèles de clignement du premier signal (S_{Q,1a}) de variation de charge électrostatique et pour chaque paire de clignements volontaires détectés et consécutifs, d'une distance temporelle de clignement volontaire respective entre lesdits clignements volontaires de la paire de clignements volontaires ;
- la vérification (S34), par l'unité (21, 15) de commande et pour chaque distance temporelle de clignement volontaire, si oui ou non ladite distance temporelle de clignement volontaire est inférieure à une distance seuil de clignement volontaire ;
- si la distance temporelle de clignement volontaire est inférieure à la distance seuil de clignement volontaire, la détection (S36) d'une troisième activité du premier œil (30) indiquant un double clignement volontaire du premier œil (30) ;
- si la distance temporelle de clignement volontaire n'est pas inférieure à la distance seuil de clignement volontaire, la détection (S38) d'une quatrième activité du premier œil (30) indiquant un seul clignement volontaire du premier œil (30).

4. Procédé (50) de détection selon la revendication 2 ou les revendications 2 et 3, dans lequel les lunettes (10) comprennent un premier verre (14a) orienté vers le premier œil (30),
dans lequel les lunettes (10) comprennent un deuxième capteur (20c) de variation de charge électrostatique comportant des troisième (22c) et quatrième (22d) électrodes espacées l'une de l'autre, orientées vers le premier œil (30) de l'utilisateur et configurées pour détecter des variations de charge électrostatique respectives générées par un mouvement d'un globe oculaire du premier œil (30) en l'absence de clignement du premier œil (30), les première (22a) et deuxième (22b) électrodes étant radialement externes par rapport aux troisième (22c) et quatrième (22d) électrodes par rapport à un centre du premier verre (14a),
dans lequel l'unité (21, 15) de commande est en outre couplée aux troisième (22c) et quatrième (22d) électrodes du deuxième capteur (20c) de variation de charge électrostatique,
dans lequel le procédé (50) de détection comprend en outre :
- l'acquisition (S10-S14), à partir de l'unité (21, 15) de commande et par le biais des troisième (22c) et quatrième (22d) électrodes du deuxième capteur (20c) de variation de charge électrostatique, d'un second signal (S_{Q,2a}) de variation de charge électrostatique indiquant une différence entre les variations de charge électrostatique détectées par les troisième (22c) et quatrième (22d) électrodes du deuxième capteur (20c) de variation de charge électrostatique ;
- si la première condition est vérifiée, la vérification (S40a), par l'unité (21, 15) de commande, de la présence d'un ou de plusieurs pics uniques dans le second signal (S_{Q,2a}) de variation de charge électrostatique, chaque pic unique étant un pic respectif du second signal (S_{Q,2a}) de variation de charge électrostatique, indiquant un mouvement respectif du globe oculaire du premier œil (30) en l'absence de clignement du premier œil (30) ;
- si lesdits un ou plusieurs pics uniques ne sont pas présents dans le second signal (S_{Q,2a}) de variation de charge électrostatique, la détermination (S24) du paramètre IEBI ;
- si lesdits un ou plusieurs pics uniques sont présents dans le second signal (S_{Q,2a}) de variation de charge électrostatique, la détermination (S40b) de l'orientation de chacun desdits pics uniques par rapport à une ligne de base du second signal (S_{Q,2a}) de variation de charge électrostatique ;
- pour chacun desdits un ou plusieurs pics uniques dans le second signal (S_{Q,2a}) de variation de charge électrostatique, la vérification (S40c) si oui ou non l'orientation est une orientation prédéfinie ;
- pour chacun desdits un ou plusieurs pics uniques dans le second signal (S_{Q,2a}) de variation de charge électrostatique, si l'orientation respective est l'orientation prédéfinie, la détection (S40d) d'une cinquième activité du premier œil (30), indiquant un premier mouvement du premier œil (30) ;
- pour chacun desdits un ou plusieurs pics uniques dans le second signal (S_{Q,2a}) de variation de charge électrostatique, si l'orientation respective n'est pas l'orientation prédéfinie, la détection (S40e) d'une sixième activité du premier œil (30), indiquant un second mouvement du premier œil (30) opposé au premier mouvement.

5. Procédé de détection (50) selon l'une quelconque des revendications précédentes, dans lequel l'étape de vérification (S16, S18) de la présence desdits un ou plusieurs schémas de clignement dans le premier signal (S_{Q,1a}) de variation de charge électrostatique comprend l'identification, pour chaque schéma de clignement, d'un premier pic et d'un second pic dans une période de clignement, le premier pic et le second pic présentent des signes opposés l'un à l'autre par rapport à une ligne de base du second signal (S_{Q,2a}) de variation de charge électrostatique et la formation dudit schéma de clignement.

6. Procédé (50) de détection selon l'une quelconque des revendications précédentes, dans lequel l'étape de détection du clignement involontaire comprend la génération d'un signal de clignement avec une première valeur indiquant la détection de clignement involontaire, et
dans lequel l'étape de détection du clignement volontaire comprend la génération du signal de clignement avec une seconde valeur indiquant la détection de clignement volontaire.

7. Procédé (50) de détection selon l'une quelconque des revendications précédentes, dans lequel l'étape (S10-S14) d'acquisition du premier signal (S_{Q,1a}) de variation de charge électrostatique comprend :
- l'acquisition de signaux (S_{R}) de détection respectifs par le biais des première (22a) et deuxième (22b) électrodes, chaque signal (S_{R}) de détection indiquant les variations de charge électrostatique sur la première (22a) ou deuxième (22b) électrode respective ;
- le calcul d'une différence entre lesdits signaux (S_{R}) de détection,
et comprend au moins l'un des éléments suivants :
- le filtrage de la différence entre lesdits signaux (S_{R}) de détection ; et
- la suppression d'un décalage de la différence entre lesdits signaux (S_{R}) de détection.

8. Procédé (50) de détection selon l'une quelconque des revendications précédentes, dans lequel les lunettes (10) comprennent en outre au moins un accéléromètre et au moins un gyroscope couplés à l'unité (21, 15) de commande, et dans lequel le procédé (50) de détection comprend en outre :
- l'acquisition (S03), par l'unité (21, 15) de commande et par le biais de l'au moins un gyroscope, d'un ou de plusieurs signaux (Sω) de vitesse angulaire indiquant des vitesses angulaires respectives corrélées à un mouvement de la tête d'un utilisateur ;
- l'acquisition (S05), par l'unité (21, 15) de commande et par le biais de l'au moins un accéléromètre, d'un ou de plusieurs signaux (S_{acc}) d'accélération linéaire indiquant des accélérations linéaires respectives corrélées au mouvement de la tête de l'utilisateur ;
- la vérification (S07), par l'unité (21, 15) de commande et en fonction des un ou plusieurs signaux (Sω) de vitesse angulaire et des un ou plusieurs signaux (S_{acc}) d'accélération linéaire, si oui ou non ledit mouvement de la tête de l'utilisateur est présent.

9. Procédé (50) de détection selon l'une quelconque des revendications précédentes lorsqu'il dépend de la revendication 2, dans lequel, si la première condition n'est pas vérifiée, l'étape de détermination (S24) du paramètre IEBI comprend le calcul d'une distance temporelle de clignement involontaire entre deux modèles de clignement consécutifs du premier signal (S_{Q,1a}) de variation de charge électrostatique , chaque modèle de clignement définissant un clignement involontaire respectif.

10. Procédé (50) de détection selon la revendication 2, dans lequel, si la première condition n'est pas vérifiée, l'étape de détermination (S24) du paramètre IEBI comprend le calcul d'une distance temporelle de clignement involontaire entre deux paires consécutives de modèles de clignement du premier signal (S_{Q,1a}) de variation de charge électrostatique et du premier signal (S_{Q,1b}) de variation de charge électrostatique supplémentaire, chaque paire de modèles de clignement du premier signal (S_{Q,1a}) de variation de charge électrostatique et du premier signal (S_{Q,1b}) de variation de charge électrostatique supplémentaire définissant un clignement involontaire respectif.

11. Procédé (50) de détection selon l'une quelconque des revendications précédentes, comprenant en outre l'étape de commande, en fonction de l'activité détectée du premier œil (30), d'une ou de plusieurs fonctionnalités des lunettes (10) ou d'un appareil couplé de manière opérationnelle aux lunettes (10).

12. Lunettes (10) pouvant être portées par un utilisateur et comprenant :
un premier capteur (20a) de variation de charge électrostatique comportant une première (22a) et une deuxième (22b) électrodes espacées l'une de l'autre, orientées vers un premier œil (30) de l'utilisateur et configurées pour détecter des variations de charge électrostatique respectives générées par un clignement du premier œil (30), et
une unité (21, 15) de commande couplée aux première (22a) et deuxième (22b) électrodes du premier capteur (20a) de variation de charge électrostatique et configurée pour :
- acquérir (S10-S14), par le biais des première (22a) et deuxième (22b) électrodes du premier capteur (20a) de variation de charge électrostatique, un premier signal (S_{Q,1a}) de variation de charge électrostatique indiquant une différence entre les variations de charge électrostatique détectées par les première (22a) et deuxième (22b) électrodes du premier capteur (20a) de variation de charge électrostatique ;
- vérifier (S16, S18) la présence d'un ou de plusieurs schémas de clignement dans le premier signal (S_{Q,1a}) de variation de charge électrostatique, chaque schéma de clignement indiquant un clignement volontaire respectif ou un clignotement involontaire respectif du premier œil (30) ;
- si le premier signal de variation de charge électrostatique (S_{Q,1a}) présente lesdits un ou plusieurs schémas de clignement, déterminer (S20, S22 ; S20', S22) pour chaque schéma de clignement si oui ou non une première condition est vérifiée, la première condition étant vérifiée si ledit schéma de clignement indique un clignement volontaire respectif ;
- si la première condition n'est pas vérifiée, détecter un clignement involontaire respectif ;
- si la première condition est vérifiée, détecter un clignement volontaire respectif,
dans lequel les lunettes (10) comprennent en outre un autre premier capteur (20b) de variation de charge électrostatique comportant des première (22a) et deuxième (22b) électrodes respectives espacées l'une de l'autre, orientées vers un second œil (30) de l'utilisateur et configurées pour détecter des variations de charge électrostatique respectives générées par un clignement du second œil (30),
l'unité (21, 15) de commande étant en outre couplée aux première (22a) et deuxième (22b) électrodes du premier capteur (20b) de variation de charge électrostatique supplémentaire et étant en outre configurée pour :
- acquérir (S10-S14), par le biais des première (22a) et deuxième (22b) électrodes du premier capteur (20b) de variation de charge électrostatique supplémentaire, un premier signal (S_{Q,1b}) de variation de charge électrostatique supplémentaire indiquant une différence entre les variations de charge électrostatique détectées par les première (22a) et deuxième (22b) électrodes du premier capteur (20b) de variation de charge électrostatique supplémentaire ; et
- vérifier (S16, S18) la présence d'un ou de plusieurs schémas de clignement respectifs dans l'autre premier signal (S_{Q,1b}) de variation de charge électrostatique, dans lequel la première condition est vérifiée si, pour chaque paire de modèles de clignement consécutifs du premier signal (S_{Q,1b}) de variation de charge électrostatique et/ou du premier signal (S_{Q,1b}) de variation de charge électrostatique supplémentaire, une distance temporelle relative entre les modèles de clignement de ladite paire de modèles de clignement est supérieure à une première période seuil.

13. Lunettes (10) selon la revendication 12, comprenant en outre un deuxième capteur (20c) de variation de charge électrostatique comportant des troisième (22c) et quatrième (22d) électrodes espacées l'une de l'autre et par rapport aux première (22a) et deuxième (22b) électrodes, orientées vers le premier œil (30) de l'utilisateur et configurées pour détecter des variations de charge électrostatique respectives générées par un mouvement d'un globe oculaire du premier œil (30) en l'absence de clignement du premier œil (30), les première (22a) et deuxième (22b) électrodes du premier capteur (20a) de variation de charge électrostatique étant radialement externes par rapport aux troisième (22c) et quatrième (22d) électrodes du deuxième capteur (20c) de variation de charge électrostatique par rapport à un centre du premier verre (14a), et
dans lequel l'unité (21, 15) de commande est en outre couplée aux troisième (22c) et quatrième (22d) électrodes du second capteur (20c) de variation de charge électrostatique.

14. Lunettes (10) selon la revendication 13, comprenant en outre un troisième capteur de variation de charge électrostatique comportant des cinquième et sixième électrodes qui sont espacées l'une de l'autre et par rapport aux première (22a), deuxième (22b), troisième (22c) et quatrième (22d) électrodes, sont orientées vers le premier œil (30) de l'utilisateur et sont configurées pour détecter des variations de charge électrostatique respectives générées par le mouvement du globe oculaire du premier œil (30) en l'absence de clignement du premier œil (30), les cinquième et sixième électrodes étant radialement internes par rapport aux première (22a) et deuxième (22b) électrodes par rapport au centre du premier verre (14a) et étant tournées angulairement par rapport aux troisième (22c) et quatrième (22d) électrodes autour du centre du premier verre (14a), et
dans lequel l'unité (21, 15) de commande est en outre couplée aux cinquième et sixième électrodes du troisième capteur de variation de charge électrostatique.

15. Produit-programme informatique pouvant être chargé dans une unité (21, 15) de commande des lunettes selon la revendication 12, ledit programme informatique étant conçu de manière à, lorsqu'il est exécuté par les lunettes selon la revendication 12, exécuter les étapes du procédé (50) de détection selon l'une quelconque des revendications 1 à 11.
